# EUROPEAN PATENT APPLICATION

(11) **EP 4 635 950 A1**
(43) Date of publication of application: **22.10.2025**
(21) Application number: 23902638.8
(22) Date of filing: 11.12.2023
(51) Int. Cl.: C07D 405/14, C07D 235/14, A61K 31/4545, A61K 31/444, A61P 3/10, A61P 3/06

(54) **NOVEL CRYSTAL FORM OF GLP-1 RECEPTOR AGONIST, METHOD FOR PREPARING SAME, PHARMACEUTICAL COMPOSITION THEREOF, AND USE THEREOF**

(30) Priority: 13.12.2022 CN 202211600175
(71) Applicant: Hangzhou Zhongmeihuadong Pharmaceutical Co., Ltd., GongShu District HangZhou, Zhejiang 310011 (CN)
(72) Inventor: ZHANG, Zhimin, Hangzhou, Zhejiang 310011 (CN); HU, Fan, Hangzhou, Zhejiang 310011 (CN); SHEN, Yurun, Hangzhou, Zhejiang 310011 (CN); PAN, Hao, Hangzhou, Zhejiang 310011 (CN); LIU, Dongzhou, Hangzhou, Zhejiang 310011 (CN)
(74) Representative: Nederlandsch Octrooibureau
(86) International application number: PCT/CN2023/137809
(87) International publication number: WO 2024/125442

(57) **Abstract**

Provided are a novel crystal form of a GLP-1 receptor agonist, a method for preparing same, a pharmaceutical composition thereof, and use thereof. The GLP-1 receptor agonist is represented by compound I, i.e., (S)-2-((4-(6-((2-fluoro-4-(oxetan-3-yl)benzyl)oxy)pyridin-2-yl)piperidin-1-yl)methyl)-1-(oxetan-2-ylmethyl)-1H-benzo[d]imidazole-6-carboxylic acid.

## Description

### Technical Field

This application relates to novel crystal forms of a GLP-1 receptor agonist and methods for preparing the same, and also relates to pharmaceutical compositions containing the crystal forms, as well as the use of the crystal forms and pharmaceutical compositions for the treatment and/or prevention of GLP-1 receptor mediated diseases or related disorders.

### Background art

Diabetes is a chronic comprehensive disease caused by absolute or relative deficiency of insulin, or reduced sensitivity of target cells to insulin, which is mainly characterized by glucose metabolic disorder. It is divided into type I diabetes and type II diabetes. Among them, type II diabetes is an adult-onset diabetes, which is an endocrine disease caused by insulin resistance and/or defects in insulin secretion, and mainly manifested by chronic elevation of blood glucose. Type II diabetes patients account for more than 90% of diabetes patients.

Insulin and GLP-1 receptor agonists are among the most effective drugs for the treatment of type II diabetes. Insulin preparations remain the most widely used drugs for diabetes globally, and about 30-40% of type II diabetes patients ultimately need to use insulin. GLP-1 preparations mainly include exenatide, liraglutide, semaglutide, etc. However, insulin preparations and GLP-1 preparations are basically polypeptide drugs and injection preparations at present. Even for oral semaglutide, there are still many restrictions in medication. Therefore, it is still necessary to further develop small molecule GLP-1 receptor agonist drugs.

Other disorders related to type II diabetes include diabetic nephropathy, diabetic ocular complications (diabetic retinopathy, uveitis related to diabetes, diabetic cataract), diabetic foot, diabetic cardiovascular complications, diabetic cerebrovascular disease, diabetic neuropathy, obesity, and hypertension.

As highly potential drugs, most of GLP-1 receptor agonists currently on the market are administered in the form of injection. Developing oral small molecule GLP-1 receptor agonists can improve patient compliance and is a development trend of GLP-1 receptor agonists.

### Summary of the Invention

(S)-2-((4-(6-((2-fluoro-4-(oxetan-3-yl)benzyl)oxy)pyridin-2-yl)piperidin-1-yl)methyl)-1-(oxetan-2-ylmethyl)-1H-benzo[d]imidazole-6-carboxylic acid (C₃₃H₃₅FN₄O₅), also referred to as Compound I in the specification and claims of the present application, is a small molecule GLP-1 receptor agonist and has the following structure:

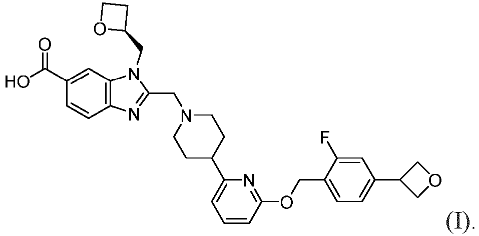

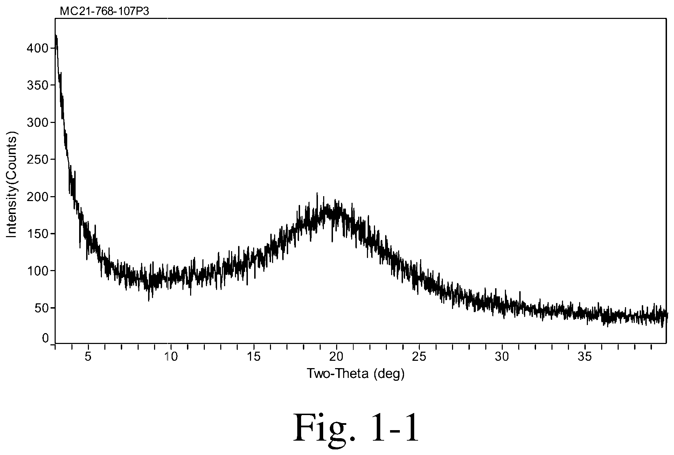

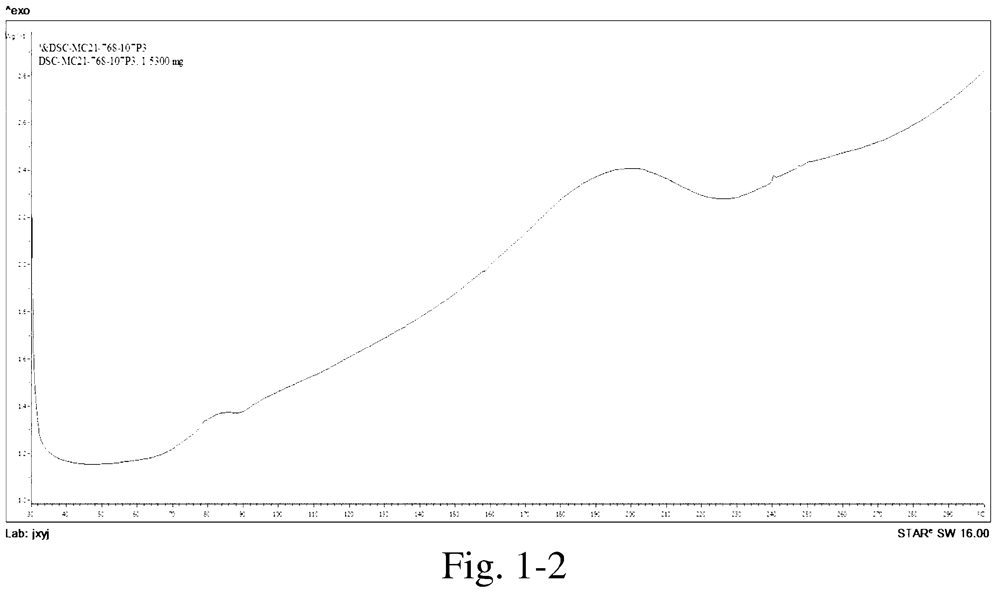

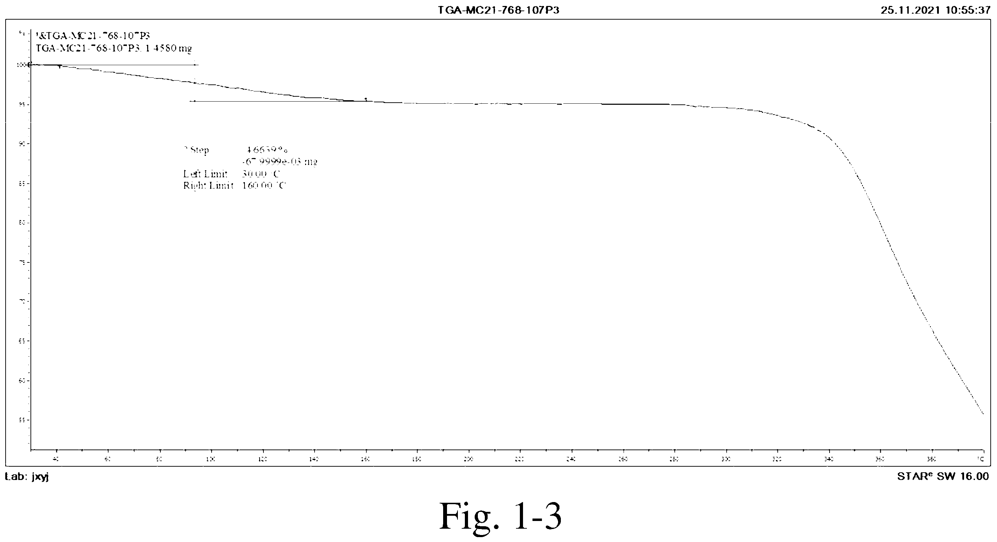

The inventors have prepared Compound I, and shown though researches that:
(1) it is in an amorphous form with an X-ray powder diffraction (XRPD) pattern as shown in Figure 1-1;
(2) no melting point was detected until the temperature was raised to 300 °C in a differential scanning calorimetry (DSC) test, with a DSC pattern as shown in Figure 1-2; and
(3) a weight loss of 4.66% was observed when the temperature was raised to 160 °C in a thermogravimetric analysis (TGA) test, with a TGA pattern as shown in Figure 1-3.

One object of the present invention is to further provide novel crystal forms of Compound I.

Therefore, in one aspect, the present invention provides crystalline forms of Compound I, including Crystal Form A, Crystal Form B, Crystal Form C, Crystal Form D, Crystal Form E, Crystal Form F, Crystal Form G, Crystal Form H, Crystal Form Ix, Crystal Form J, Crystal Form K, Crystal Form L, Crystal Form M, Crystal Form N, Crystal Form O, and Crystal Form P, preferably Crystal Form A and Crystal Form G, and more preferably Crystal Form G, as described below.

The crystalline forms of Compound I of the present invention have good physical and chemical properties, such as low hygroscopicity, good physicochemical stability (thermodynamic stability, solid-state stability, high-temperature resistance, high-humidity resistance, and/or high-pressure resistance), and potentially have good drugability and/or bioavailability.

In another aspect, the present invention provides methods for preparing the crystalline forms of Compound I of the present invention.

In another aspect, the present invention provides a pharmaceutical composition, which contains the crystalline form of Compound I of the present invention, preferably the Crystal Form A or Crystal Form G, and more preferably the Crystal Form G.

In another aspect, the present invention provides the crystalline forms of Compound I of the present invention, preferably the Crystal Form A or Crystal Form G, more preferably the Crystal Form G, or the pharmaceutical composition of the present invention, for use in the treatment and/or prevention of GLP-1 receptor mediated diseases and related disorders.

In another aspect, the present invention provides the use of the crystalline forms of Compound I of the present invention, preferably the Crystal Form A or Crystal Form G, more preferably the Crystal Form G, or the pharmaceutical composition of the present invention, in the manufacture of a medicament for the treatment and/or prevention of GLP-1 receptor mediated diseases or related disorders.

In another aspect, the present invention provides a method for treating and/or preventing GLP-1 receptor mediated diseases or related disorders, which comprises administering an effective amount of the crystalline form of Compound I of the present invention, preferably the Crystal Form A or Crystal Form G, more preferably the Crystal Form G, or the pharmaceutical composition of the present invention to a subject in need thereof.

In some embodiments, the GLP-1 receptor mediated diseases or related disorders are selected from diabetes, hyperglycemia, insulin resistance, impaired glucose tolerance, diabetic nephropathy, diabetic neuropathy, diabetic retinopathy, adipocyte dysfunction, obesity, non-alcoholic fatty liver disease, dyslipidemia, and hyperinsulinemia. In some embodiments, the diabetes is selected from type I diabetes (T1D) and/or type II diabetes (T2DM), idiopathic T1D, early-onset T2DM, latent autoimmune diabetes, atypical diabetes in adolescents, and gestational diabetes.

### Description of Drawings

Figure 1-1 is the XRPD pattern of the amorphous form of Compound I; Figure 1-2 is the DSC pattern of the amorphous form; Figure 1-3 is the TGA pattern of the amorphous form.
Figure 2-1 is the XRPD pattern of the Crystal Form A of Compound I; Figure 2-2 is the DSC pattern of the Crystal Form A; Figure 2-3 is the TGA pattern of the Crystal Form A; Figure 2-4 is the ¹H NMR spectrum of the Crystal Form A; Figure 2-5 is the DVS pattern of the Crystal Form A.
Figure 3-1 is the XRPD pattern of the Crystal Form G of Compound I; Figure 3-2 is the DSC pattern of the Crystal Form G; Figure 3-3 is the TGA pattern of the Crystal Form G; Figure 3-4 is the ¹H NMR spectrum of the Crystal Form G; Figure 3-5 is the DVS pattern of the Crystal Form G.
Figure 4-1 is the XRPD pattern of the Crystal Form C of Compound I; Figure 4-2 is the DSC pattern of the Crystal Form C; Figure 4-3 is the TGA pattern of the Crystal Form C; Figure 4-4 is the ¹H NMR spectrum of the Crystal Form C.
Figure 5-1 is the XRPD pattern of the Crystal Form D of Compound I; Figure 5-2 is the DSC pattern of the Crystal Form D; Figure 5-3 is the TGA pattern of the Crystal Form D; Figure 5-4 is the ¹H NMR spectrum of the Crystal Form D.
Figure 6-1 is the XRPD pattern of the Crystal Form E of Compound I; Figure 6-2 is the DSC pattern of the Crystal Form E; Figure 6-3 is the TGA pattern of the Crystal Form E; Figure 6-4 is the ¹H NMR spectrum of the Crystal Form E.
Figure 7-1 is the XRPD pattern of the Crystal Form Ix of Compound I; Figure 7-2 is the DSC pattern of the Crystal Form Ix; Figure 7-3 is the TGA pattern of the Crystal Form Ix; Figure 7-4 is the ¹H NMR spectrum of the Crystal Form Ix.
Figure 8-1 is the XRPD pattern of the Crystal Form J of Compound I; Figure 8-2 is the DSC pattern of the Crystal Form J; Figure 8-3 is the TGA pattern of the Crystal Form J; Figure 8-4 is the ¹H NMR spectrum of the Crystal Form J.
Figure 9-1 is the XRPD pattern of the Crystal Form K of Compound I; Figure 9-2 is the DSC pattern of the Crystal Form K; Figure 9-3 is the TGA pattern of the Crystal Form K; Figure 9-4 is the ¹H NMR spectrum of the Crystal Form K.
Figure 10-1 is the XRPD pattern of the Crystal Form L of Compound I; Figure 10-2 is the DSC pattern of the Crystal Form L; Figure 10-3 is the TGA pattern of the Crystal Form L; Figure 10-4 is the ¹H NMR spectrum of the Crystal Form L.
Figure 11-1 is the XRPD pattern of the Crystal Form M of Compound I; Figure 11-2 is the DSC pattern of the Crystal Form M; Figure 11-3 is the TGA pattern of the Crystal Form M; Figure 11-4 is the ¹H NMR spectrum of the Crystal Form M.
Figure 12-1 is the XRPD pattern of the Crystal Form N of Compound I; Figure 12-2 is the DSC pattern of the Crystal Form N; Figure 12-3 is the TGA pattern of the Crystal Form N; Figure 12-4 is the ¹H NMR spectrum of the Crystal Form N.
Figure 13-1 is the XRPD pattern of the Crystal Form O of Compound I; Figure 13-2 is the DSC pattern of the Crystal Form O; Figure 13-3 is the TGA pattern of the Crystal Form O; Figure 13-4 is the ¹H NMR spectrum of the Crystal Form O.
Figure 14 is the XRPD pattern of the Crystal Form B of Compound I.
Figure 15 is the XRPD pattern of the Crystal Form H of Compound I.
Figure 16 is the XRPD pattern of the Crystal Form F of Compound I.
Figure 17 is the XRPD pattern of the Crystal Form P of Compound I.

### Detailed Description of Invention

The present invention is further explained and illustrated below. It should be understood that the terms are for descriptive purpose, instead of imposing any limitation on the present invention.

### Definitions

Unless otherwise specified, all technical and scientific terms used herein have the same meaning as commonly understood by those skilled in the art to which the present invention pertains. In case of any discrepancy, the definitions herein shall prevail. When an amount, concentration, or other values or parameters are expressed in the form of a range, a preferred range, or a preferred upper limit numerical value and a preferred lower limit numerical value, it should be understood to be equivalent to specifically disclosing any ranges obtained by combining any pair of an upper limit numerical value or a preferred numerical value of a range with any lower limit numerical value or a preferred numerical value of the range. Unless otherwise specified, a numerical range listed herein is intended to include the endpoints of the range and all integers and fractions (decimals) within the range.

The term "about", when used in conjunction with a numerical variable, generally means that the numerical value of the variable and all numerical values of the variable are within the experimental error (for example, within a 95% confidence interval for the mean value) or within ±20%, ±10%, ±5%, or ±2% of the specified numerical value.

As used herein, the term "about", when describing the diffraction angles of XRPD, means being within the acceptable standard error of the value as considered by a person of ordinary skill in the art, such as ±0.05, ±0.10, ±0.20, ±0.30, ±1, ±2, or ±3, etc.

The term "comprise" or similar synonymous expressions such as "include", "contain", and "have" are open-ended and do not exclude additional unrecited elements, steps, or components. The expression "consist of" excludes any unspecified element, step, or component. The term "substantially consist of" means that the scope is limited to the specified elements, steps, or components, with the addition of optionally existing elements, steps, or components that will not essentially affect the basic and novel characteristics of the claimed subject matter. It should be understood that the terms "comprise", "include", and similar terms encompass the terms "substantially consist of" and "consist of".

As used herein, the term "optional" or "optionally" means that the subsequently described event or situation may or may not occur, and that the description includes instances where the event or situation occurs, and instances where the event or situation does not occur.

Unless otherwise specified, all percentages, parts and the like herein are by weight.

As used herein, the term "crystal form" or "crystal" refers to any solid substance that presents three-dimensional ordering, and as opposed to an amorphous solid substance, produces a characteristic XRPD pattern with well-defined peaks.

As used herein, the term "X-ray powder diffraction pattern" or "XRPD pattern" refers to an experimentally observed diffraction pattern, or parameters, data, or values derived therefrom. An XRPD pattern is usually characterized by peak positions (abscissa) and/or peak intensities (ordinate).

As used herein, the term "diffraction angle" or "2θ" refers to the peak position expressed in degrees (°) based on X-ray diffraction experiment setting, and is usually an abscissa unit in a diffraction pattern. If the reflection is diffracted when the incident beam forms an angle θ with a certain lattice plane, it is necessary to record the reflected beam at an angle of 2θ in the experimental setting. It should be understood that the specific 2θ values mentioned herein for a specific crystal form are intended to denote the 2θ values (expressed in degrees) measured using the X-ray diffraction experimental conditions described herein. For example, as described herein, Cu-Kα (Kα1 (Å): 1.5406) monochromatic radiation is used. The XRPD patterns herein are preferably collected on a Bruker D8 Advance (Bruker, GER) X-ray powder diffraction analyzer.

As used herein, the term "substantially the same" or "substantially as shown in Figure x" for X-ray diffraction peaks means that the variations in representative peak positions and intensities are taken into account. For example, those skilled in the art will understand that the peak positions (2θ) will show some variations, usually up to 0.1 to 0.2 degrees, and the instruments used to measure diffraction will also cause some variations. In addition, those skilled in the art will understand that the relative peak intensities will vary due to inter-instrument differences, as well as the degree of crystallinity, preferred orientation, the surface of the prepared sample, and other factors known to those skilled in the art.

Similarly, as used herein, the expressions "substantially as shown in Figure x" for DSC patterns and TGA patterns are also intended to encompass the variations related to these analytical techniques known to those skilled in the art. For example, there is usually a variation of up to ±0.2 °C for well-defined peaks, and even larger (for example, up to ±1 °C) for broad peaks in a DSC pattern.

The nuclear magnetic resonance spectra in this application are preferably collected on a Bruker AVANCE-III or Bruker AVANCE NEO (Bruker, GER) nuclear magnetic resonance spectrometer. Unless otherwise specified, MeOD-d4 is used as the solvent.

As used herein, the term "solvent" or "good solvent" means a solvent in which Compound I can dissolve, or has a relatively higher solubility. As used herein, the term "anti-solvent" means a solvent in which Compound I is insoluble or substantially insoluble, or has a relatively lower solubility. In the present application, the terms "solvent" or "good solvent" and "anti-solvent" can also be relative and do not represent the absolute solubility of Compound I therein. The same solvent can act as a good solvent in some cases and as an anti-solvent in other cases.

As used herein, the numerical ranges (such as "1 to 10") and their sub-ranges (such as "2 to 10", "2 to 6", "3 to 10") contemplate any number within the numerical range (for example, 1, 2, 3, 4, 5, 6, 7, 8, 9, or 10).

As used herein, the term "room temperature" refers to 20 °C ± 5 °C.

### I. Crystalline Forms of Compound I

The present invention provides crystalline forms of Compound I, including Crystal Form A, Crystal Form B, Crystal Form C, Crystal Form D, Crystal Form E, Crystal Form F, Crystal Form G, Crystal Form H, Crystal Form Ix, Crystal Form J, Crystal Form K, Crystal Form L, Crystal Form M, Crystal Form N, Crystal Form O, and Crystal Form P, as described below. The preferred crystalline forms are the Crystal Form A and Crystal Form G, and the Crystal Form G is more preferred.

The present invention also provides methods for preparing the crystal forms, including but not limited to slow evaporation, suspending with stirring at a low-temperature (for example, 4-8 °C), suspending with stirring at room-temperature, suspending with stirring at a high-temperature (for example, 50 °C), addition of an anti-solvent, addition of an anti-anti-solvent, cooling crystallization, gas-liquid diffusion, gas-solid diffusion, water vapor stress, polymer induction, grinding, rotary evaporation, and cyclic temperature rise and fall.

### i. Crystal Form A

The present invention provides Crystal Form A of Compound I, characterized in that the X-ray powder diffraction (XRPD) pattern of the Crystal Form A includes diffraction peaks at the following diffraction angles (2θ): about 4.97 ± 0.2°, 11.74 ± 0.2°, 13.27 ± 0.2°, 21.04 ± 0.2°, and 24.06 ± 0.2°.

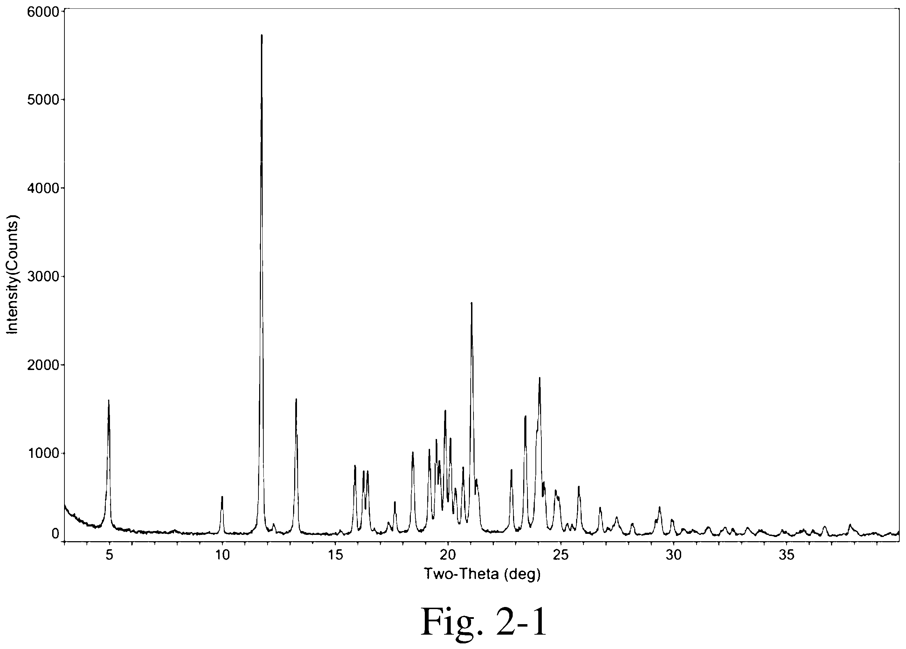

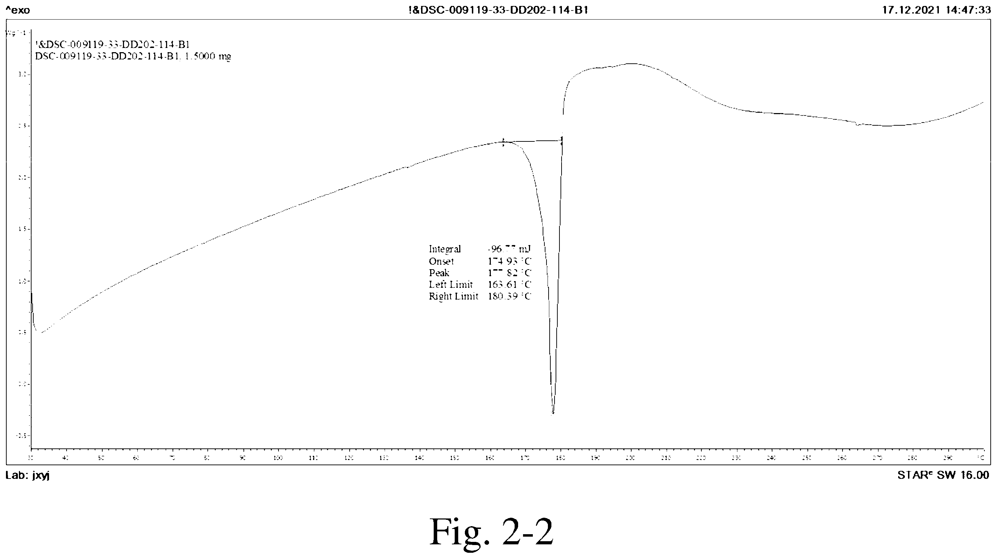

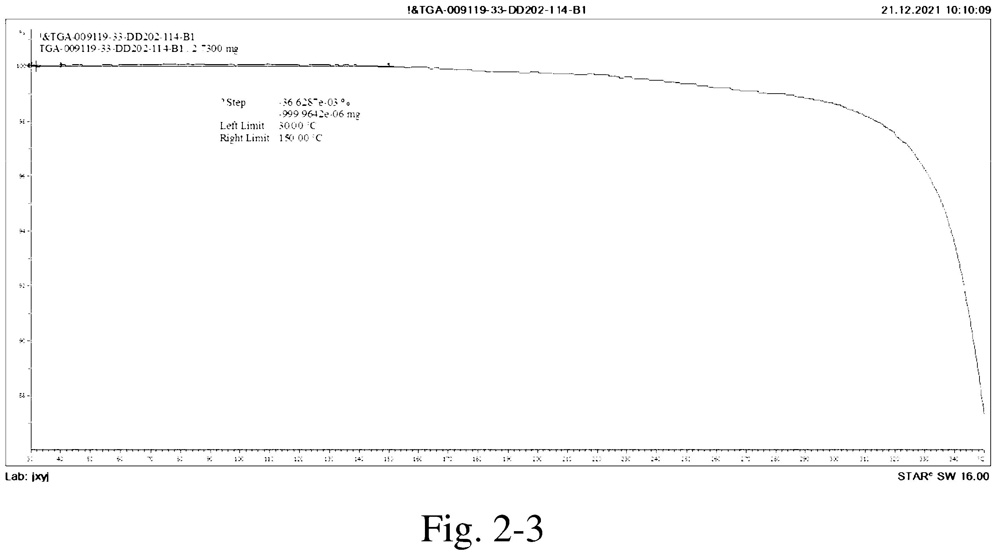

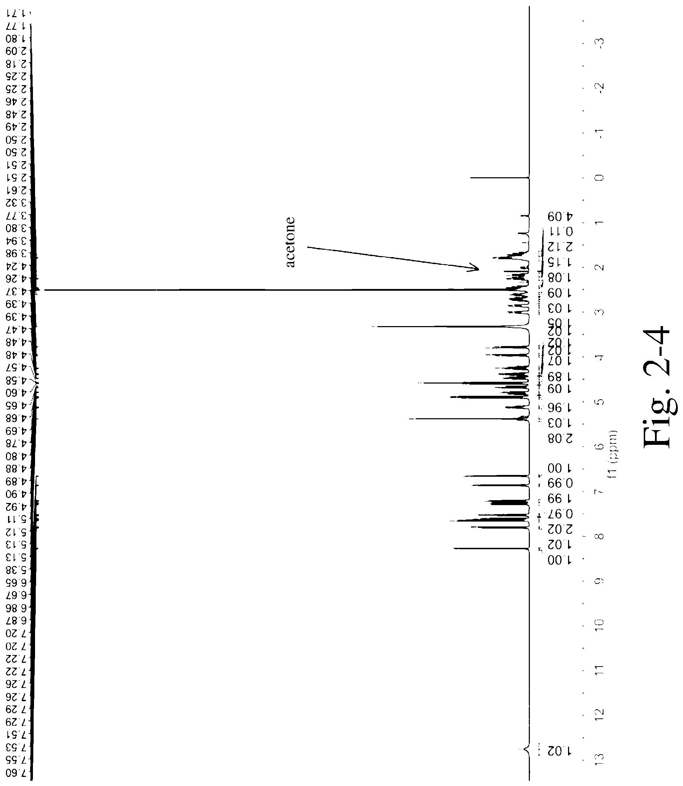

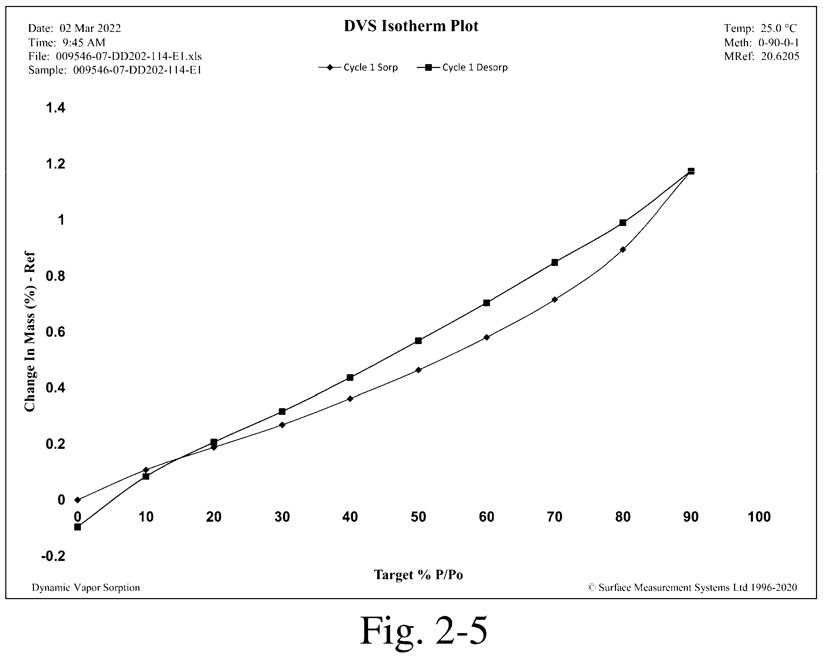

Alternatively or additionally, the Crystal Form A has any one, two, or all of the following characteristics:
(1) the differential scanning calorimetry (DSC) pattern of the Crystal Form A has one endothermic peak with a peak value at about 177.8°C ± 3.0°C;
(2) the Crystal Form A has a weight loss of about 0.037% during the process of heating to about 150°C ± 3°C, as measured by thermogravimetric analysis (TGA); and
(3) the ¹H NMR spectrum of the Crystal Form A is substantially as shown in Figure 2-4.

In some preferred embodiments, the XRPD pattern of the Crystal Form A further includes diffraction peaks at any one, more, or all of the following diffraction angles (2θ): about 15.88 ± 0.2°, 16.26 ± 0.2°, 16.44 ± 0.2°, 18.43 ± 0.2°, 19.17 ± 0.2°, 19.48 ± 0.2°, 19.65 ± 0.2°, 19.89 ± 0.2°, 20.11 ± 0.2°, 20.67 ± 0.2°, 21.27 ± 0.2°, 22.81 ± 0.2°, and 23.4 ± 0.2°.

In some preferred embodiments, the XRPD pattern of the Crystal Form A further includes diffraction peaks at any one, more, or all of the following diffraction angles (2θ): about 9.99 ± 0.2°, 17.65 ± 0.2°, 20.32 ± 0.2°, 24.26 ± 0.2°, 24.76 ± 0.2°, 25.79 ± 0.2°, 26.75 ± 0.2°, 27.47 ± 0.2°, 28.19 ± 0.2°, 29.38 ± 0.2°, and 29.91 ± 0.2°.

In some preferred embodiments, the DSC pattern of the Crystal Form A is substantially as shown in Figure 2-2.

In some preferred embodiments, the TGA pattern of the Crystal Form A is substantially as shown in Figure 2-3.

In some more preferred embodiments, the XRPD pattern of the Crystal Form A includes diffraction peaks at diffraction angles (2θ) that are substantially the same as those shown in Figure 2-1, and further more preferably, the XRPD pattern of the Crystal Form A is as shown in Figure 2-1.

The TGA weight loss of the Crystal Form A is small, and the ¹H NMR spectrum in Figure 2-4 shows no obvious solvent residue. In some embodiments, the Crystal Form A is not a solvate. More preferably, the Crystal Form A is an anhydride.

In some embodiments, the Crystal Form A has a melting point greater than about 170°C.

In some embodiments, no crystal form change is observed after the Crystal Form A is dried at about 50°C for 3 hours.

The Crystal Form A has good solid-state stability, high-temperature stability, high-humidity stability, pressure stability, and low hygroscopicity.

In another aspect, the present invention also provides a method for preparing the Crystal Form A, comprising stirring a suspension of amorphous Compound I in acetone:H₂O (for example, at about 1:4, v:v) at an elevated temperature to obtain the Crystal Form A as a solid precipitate.

In some embodiments, the elevated temperature is a temperature of about 40-70°C, such as a temperature of about 45-65°C or about 50-60°C, preferably about 50°C.

In some embodiments, the stirring can be carried out for a suitable period of time, for example, about 1-2 days.

### ii. Crystal Form G

The present invention also provides Crystal Form G of Compound I, characterized in that the XRPD pattern of the Crystal Form G includes diffraction peaks at the following diffraction angles (2θ): about 4.99 ± 0.2°, 10.05 ± 0.2°, 16.99 ± 0.2°, 19.21 ± 0.2°, and 20.28 ± 0.2°.

Alternatively or additionally, the Crystal Form G has any one, two, or all of the following characteristics:
(1) the DSC pattern of the Crystal Form G has one endothermic peak with a peak value at about 188.3°C ± 3.0°C; and
(2) the Crystal Form G has a weight loss of about 0.494% during the process of heating to about 180°C ± 3°C, as measured by TGA.

In some preferred embodiments, the XRPD pattern of the Crystal Form G further includes diffraction peaks at any one, more, or all of the following diffraction angles (2θ): about 12.33 ± 0.2°, 15.84 ± 0.2°, 17.34 ± 0.2°, 17.59 ± 0.2°, 20.67 ± 0.2°, 21.68 ± 0.2°, and 26.59 ± 0.2°.

In some preferred embodiments, the XRPD pattern of the Crystal Form G further includes diffraction peaks at any one, more, or all of the following diffraction angles (2θ): about 12.62 ± 0.2°, 15.00 ± 0.2°, 18.30 ± 0.2°, 23.88 ± 0.2°, 24.72 ± 0.2°, 29.12 ± 0.2°, and 33.76 ± 0.2°.

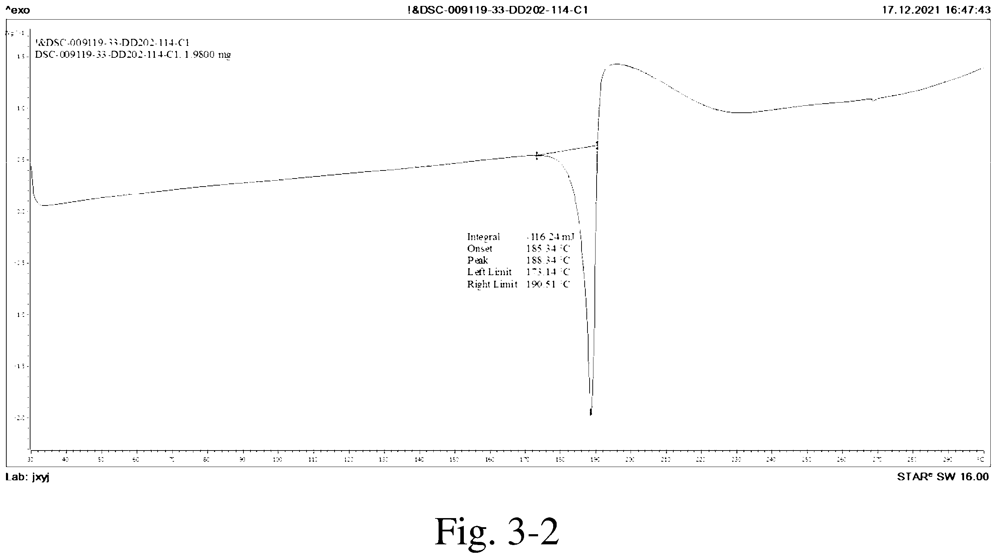

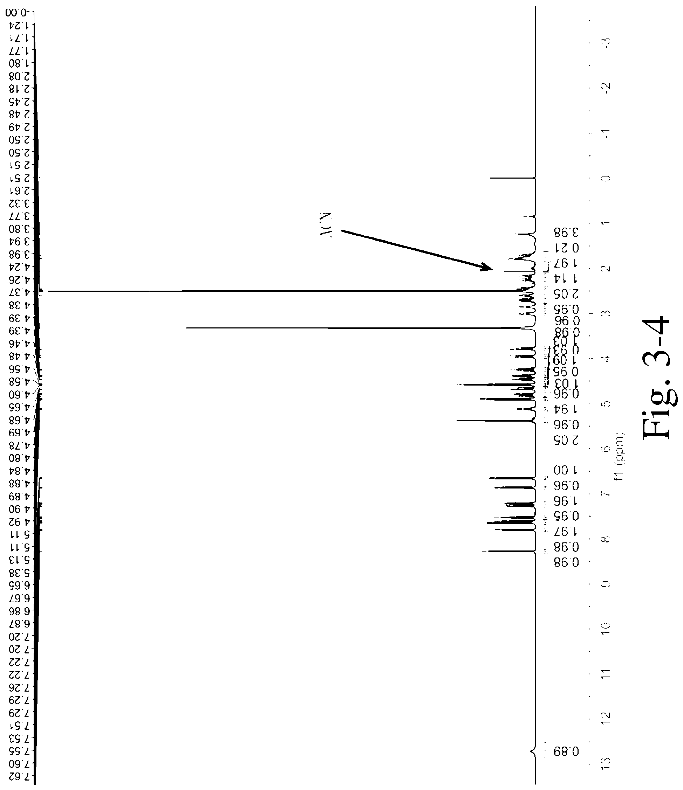

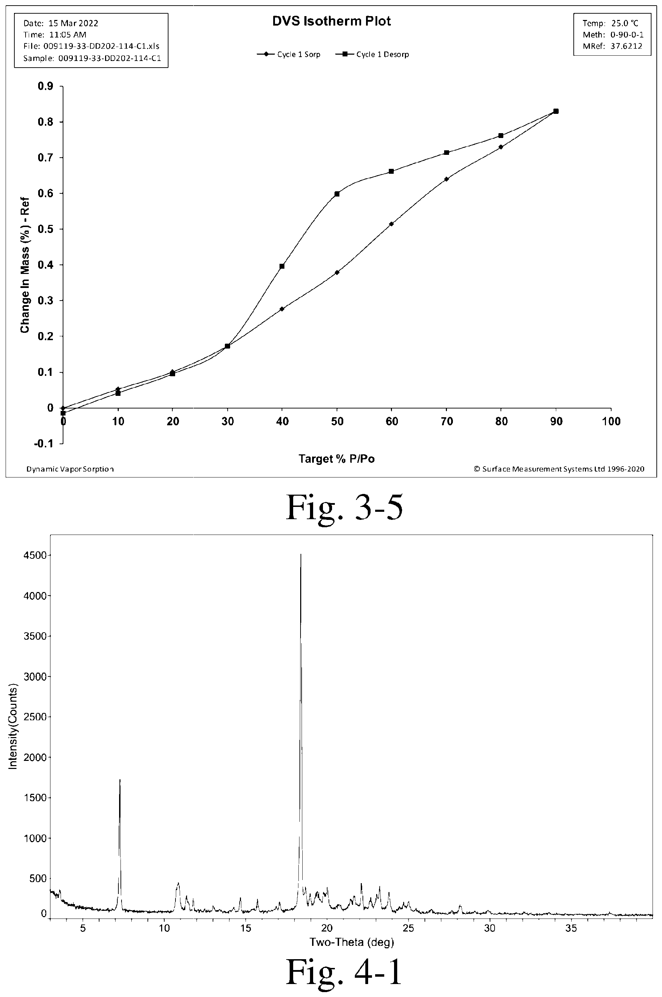

In some preferred embodiments, the DSC pattern of the Crystal Form G is substantially as shown in Figure 3-2.

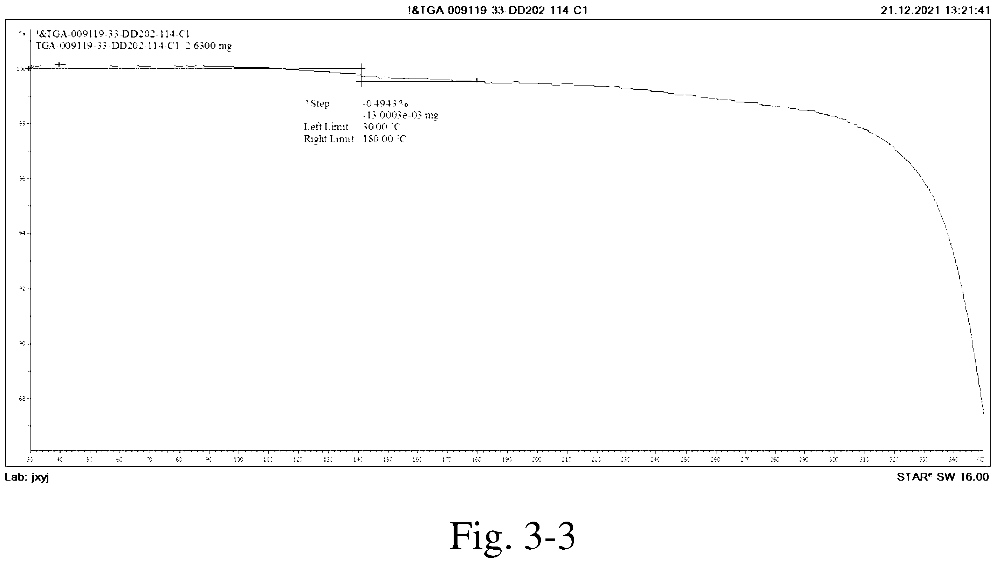

In some preferred embodiments, the TGA pattern of the Crystal Form G is substantially as shown in Figure 3-3.

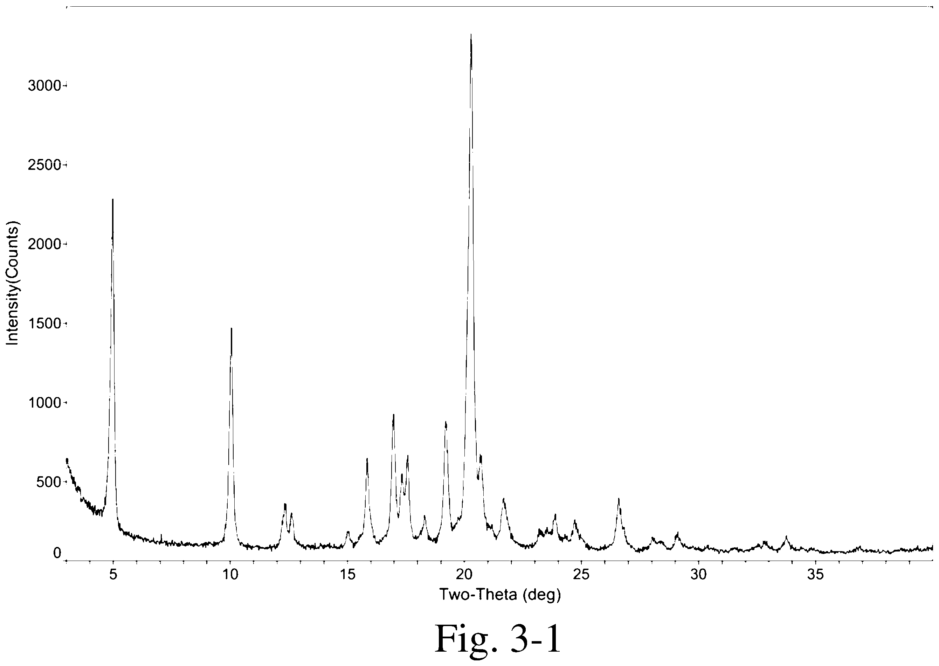

In some more preferred embodiments, the XRPD pattern of the Crystal Form G includes diffraction peaks at diffraction angles (2θ) that are substantially the same as those shown in Figure 3-1, and further more preferably, the XRPD pattern of the Crystal Form G is as shown in Figure 3-1.

The TGA weight loss of the Crystal Form G is small, and ¹H NMR analysis shows no obvious solvent residue. In some embodiments, the Crystal Form G is not a solvate. More preferably, the Crystal Form G is an anhydride.

In some embodiments, the Crystal Form G has a melting point greater than about 170°C.

The Crystal Form G is a thermodynamically more stable crystal form at room temperature and 50°C. The suspension competition tests conducted on Crystal Form A and Crystal Form G in solvents such as ethanol (EtOH) and a mixture of methanol (MeOH) and water at room temperature and 50°C show that Crystal Form A transforms into Crystal Form G, indicating that Crystal Form G is thermodynamically more stable.

In some embodiments, no crystal form change is observed after the Crystal Form G is dried at 50°C for 3 hours. The Crystal Form G also has good solid-state stability, high-temperature stability, high-humidity stability, pressure stability, and low hygroscopicity.

In another aspect, the present invention also provides a method for preparing the Crystal Form G, comprising:
(1) providing a clear solution of Compound I in acetonitrile (ACN) having a first elevated temperature; and
(2) allowing the solution to cool naturally to room temperature to obtain the Crystal Form G as a solid precipitate.

In some embodiments, the first elevated temperature is a temperature of about 50-70°C, such as a temperature of about 55-65°C or about 60-65°C, preferably about 60°C.

In some embodiments, the method comprises stirring a suspension of Compound I in acetonitrile at a second elevated temperature, and then heating the suspension to the first elevated temperature to obtain the clear solution.

In some embodiments, the second elevated temperature is a temperature of about 40-60°C, such as a temperature of about 45-55°C or about 50-55°C, preferably about 50°C.

### iii. Crystal Form C

The present invention also provides Crystal Form C of Compound I, characterized in that the XRPD pattern of the Crystal Form C includes diffraction peaks at the following diffraction angles (2θ): about 7.28 ± 0.2°, 10.87 ± 0.2°, 18.39 ± 0.2°, 22.12 ± 0.2°, and 23.24 ± 0.2°.

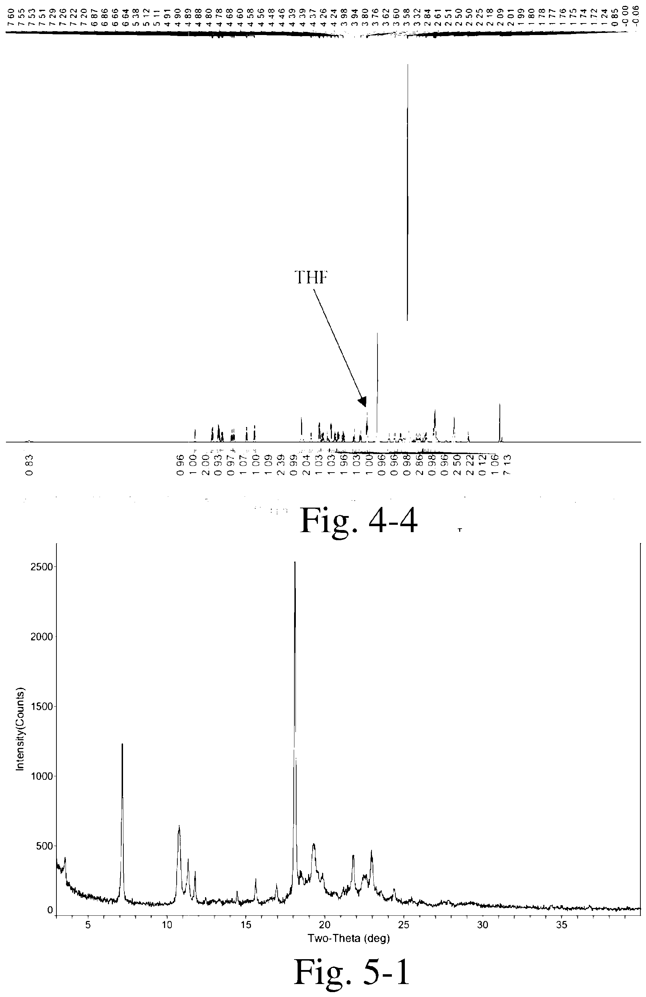

Alternatively or additionally, the Crystal Form C has any one, two, or all of the following characteristics:
(1) the DSC pattern of the Crystal Form C has one endothermic peak with a peak value at about 109.0°C ± 3.0°C;
(2) the Crystal Form C has a weight loss of about 7.81% during the process of heating to about 120°C ± 3°C, as measured by TGA; and
(3) the ¹H NMR spectrum of the Crystal Form C is substantially as shown in Figure 4-4.

In some preferred embodiments, the XRPD pattern of the Crystal Form C further includes diffraction peaks at any one, more, or all of the following diffraction angles (2θ): about 11.37 ± 0.2°, 14.67 ± 0.2°, 15.73 ± 0.2°, 18.67 ± 0.2°, 19.39 ± 0.2°, 19.79 ± 0.2°, 20.01 ± 0.2°, 21.66 ± 0.2°, and 23.80 ± 0.2°.

In some preferred embodiments, the XRPD pattern of the Crystal Form C further includes diffraction peaks at any one, more, or all of the following diffraction angles (2θ): about 11.79 ± 0.2°, 17.09 ± 0.2°, 18.96 ± 0.2°, 21.43 ± 0.2°, 22.68 ± 0.2°, 25.01 ± 0.2°, and 28.17 ± 0.2°.

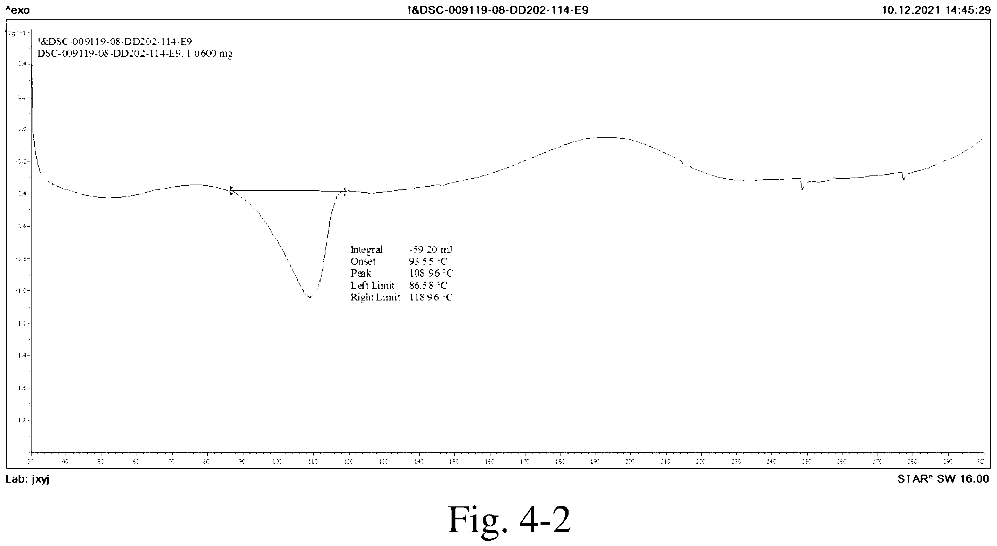

In some preferred embodiments, the DSC pattern of the Crystal Form C is substantially as shown in Figure 4-2.

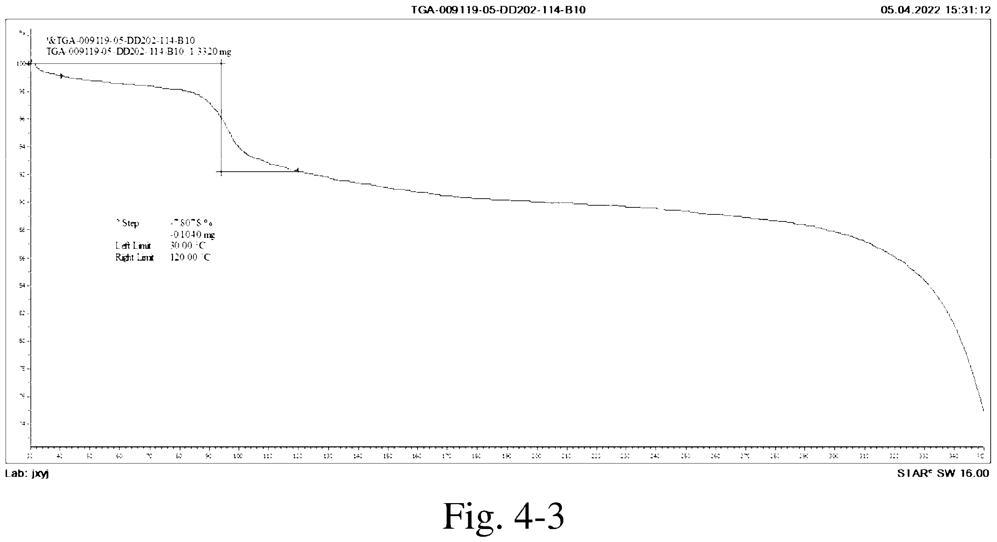

In some preferred embodiments, the TGA pattern of the Crystal Form C is substantially as shown in Figure 4-3.

In some more preferred embodiments, the XRPD pattern of the Crystal Form C includes diffraction peaks at diffraction angles (2θ) that are substantially the same as those shown in Figure 4-1, and further more preferably, the XRPD pattern of the Crystal Form C is as shown in Figure 4-1.

In some embodiments, the Crystal Form C is a solvate, more specifically a solvate with tetrahydrofuran (THF), wherein the stoichiometric ratio of the Compound I to the THF is preferably about 1:0.72.

In the DSC test, the Crystal Form C transforms into an amorphous form when heated to 110°C for 5 minutes.

In another aspect, the present invention also provides a method for preparing the Crystal Form C, comprising:
(1) providing a clear solution of Compound I in THF;
(2) adding an anti-solvent to the clear solution under stirring to obtain the Crystal Form C as a solid precipitate.

In some embodiments, the anti-solvent is water.

### iv. Crystal Form D

The present invention also provides Crystal Form D of Compound I, characterized in that the XRPD pattern of the Crystal Form D includes diffraction peaks at the following diffraction angles (2θ): about 7.17 ± 0.2°, 10.77 ± 0.2°, 11.36 ± 0.2°, 18.10 ± 0.2°, 19.29 ± 0.2°, and 2.95 ± 0.2°.

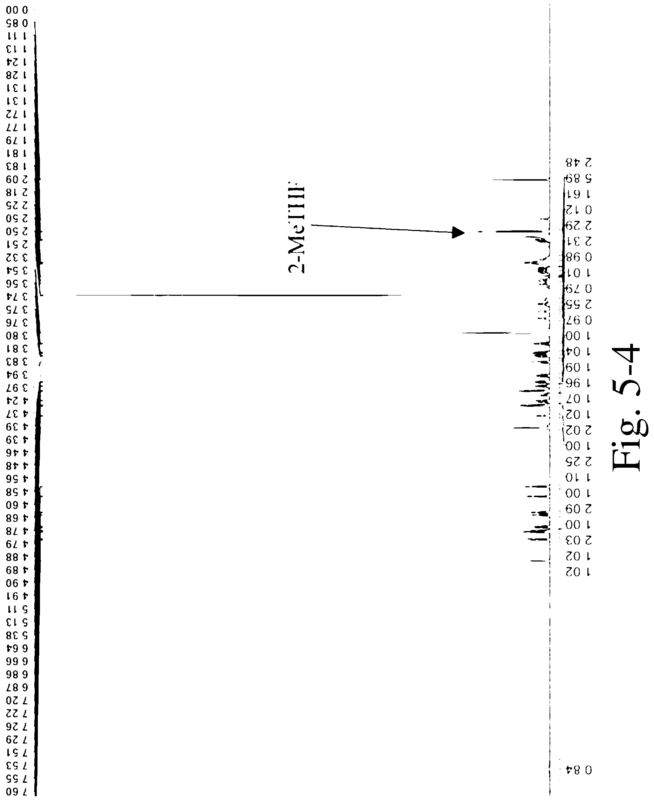

Alternatively or additionally, the Crystal Form D has any one, two, or all of the following characteristics:
(1) the DSC pattern of the Crystal Form D has one endothermic peak with a peak value at about 104.1°C ± 3.0°C;
(2) the Crystal Form D has a weight loss of about 11.75% during the process of heating to about 180°C ± 3°C, as measured by TGA; and
(3) the ¹H NMR spectrum of the Crystal Form D is substantially as shown in Figure 5-4.

In some preferred embodiments, the XRPD pattern of the Crystal Form D further includes diffraction peaks at any one, more, or all of the following diffraction angles (2θ): about 3.54 ± 0.2°, 11.78 ± 0.2°, 15.62 ± 0.2°, 18.45 ± 0.2°, and 21.78 ± 0.2°.

In some preferred embodiments, the XRPD pattern of the Crystal Form D further includes diffraction peaks at any one, more or all of the following diffraction angles (2θ): about 14.44 ± 0.2°, 16.93 ± 0.2°, 19.85 ± 0.2°, 22.44 ± 0.2°, and 4.39 ± 0.2°.

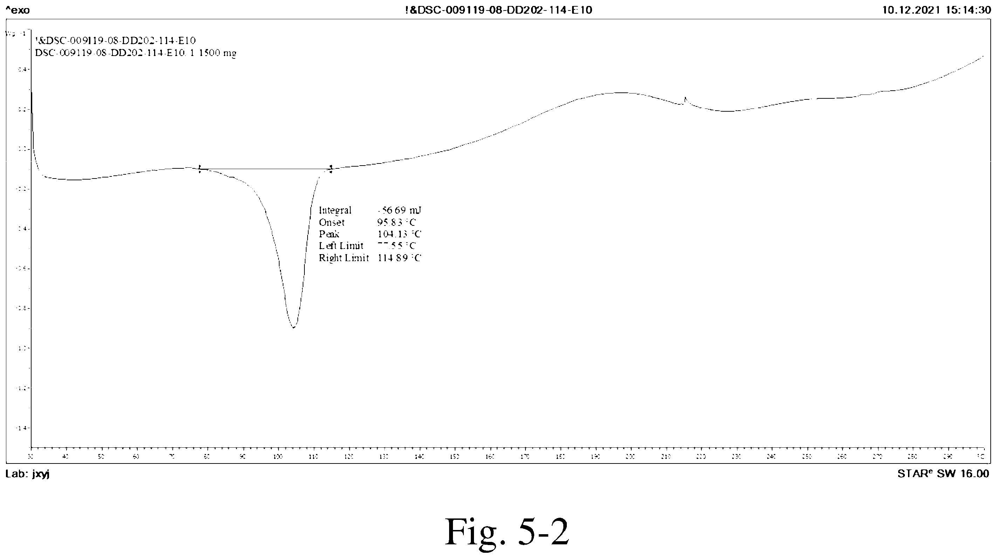

In some preferred embodiments, the DSC pattern of the Crystal Form D is substantially as shown in Figure 5-2.

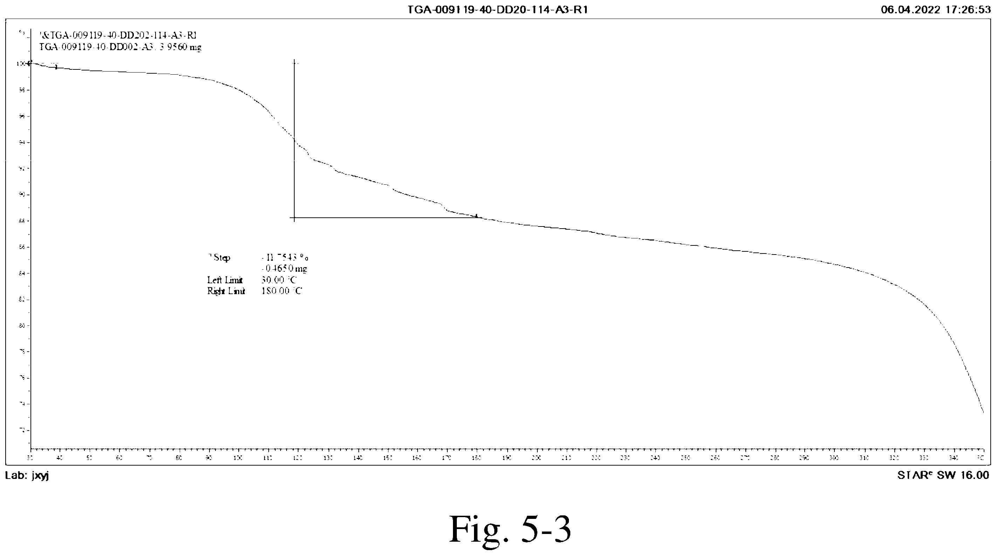

In some preferred embodiments, the TGA pattern of the Crystal Form D is substantially as shown in Figure 5-3.

In some more preferred embodiments, the XRPD pattern of the Crystal Form D includes diffraction peaks at diffraction angles (2θ) that are substantially the same as those shown in Figure 5-1, and further more preferably, the XRPD pattern of the Crystal Form D is as shown in Figure 5-1.

In some embodiments, the Crystal Form D is a solvate, more specifically a solvate with 2-methyltetrahydrofuran (2-MeTHF), wherein the stoichiometric ratio of the Compound I to the 2-MeTHF is preferably about 1:0.79.

In the DSC test, the Crystal Form D converts into an amorphous form when heated to 120 °C for 10 minutes.

In another aspect, the present invention also provides a method for preparing the Form D, comprising:
(1) providing a clear solution of Compound I in 2-MeTHF;
(2) adding an anti-solvent to the clear solution under stirring to obtain the Crystal Form D as a solid precipitate.

In some embodiments, the anti-solvent is heptane (HEP).

### v. Crystal Form E

The present invention also provides Crystal Form E of Compound I, characterized in that the XRPD pattern of the Crystal Form E includes diffraction peaks at the following diffraction angles (2θ): about 7.29 ± 0.2°, 10.52 ± 0.2°, 11.43 ± 0.2°, 11.87 ± 0.2°, and 8.37 ± 0.2°.

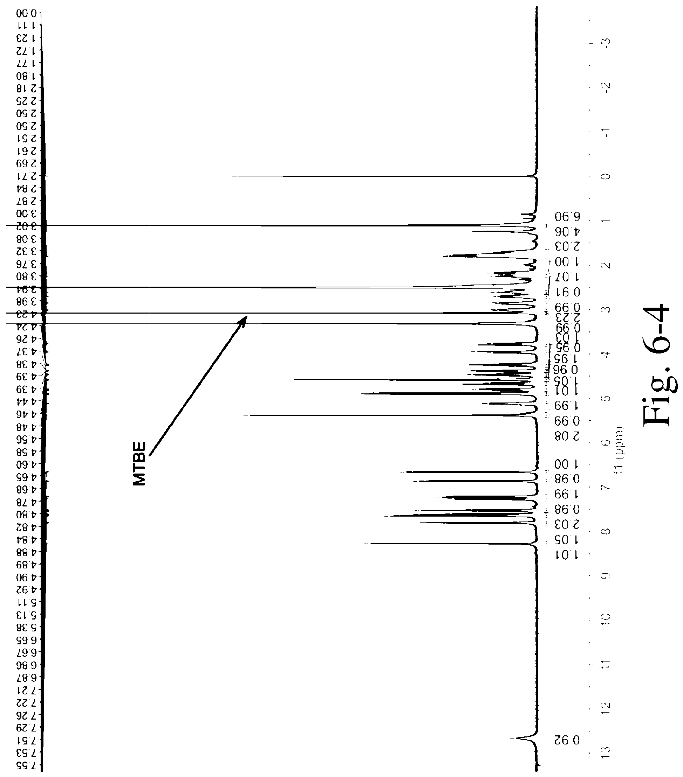

Alternatively or additionally, the Crystal Form E has any one, two, or all of the following characteristics:
(1) the DSC pattern of the Crystal Form E has one endothermic peak with a peak at about 105.6 °C ± 3.0 °C;
(2) the Crystal Form E has a weight loss of about 11.46% during the process of heating to about 140 °C ± 3 °C, as measured by TGA; and
(3) the ¹H NMR spectrum of the Crystal Form E is substantially as shown in Figure 6-4.

In some preferred embodiments, the XRPD pattern of the Crystal Form E further includes diffraction peaks at any one, more or all of the following diffraction angles (2θ): about 13.55 ± 0.2°, 14.67 ± 0.2°, 15.78 ± 0.2°, 17.13 ± 0.2°, 19.28 ± 0.2°, 20.05 ± 0.2°, 21.20 ± 0.2°, 22.08 ± 0.2°, and 24.55 ± 0.2°.

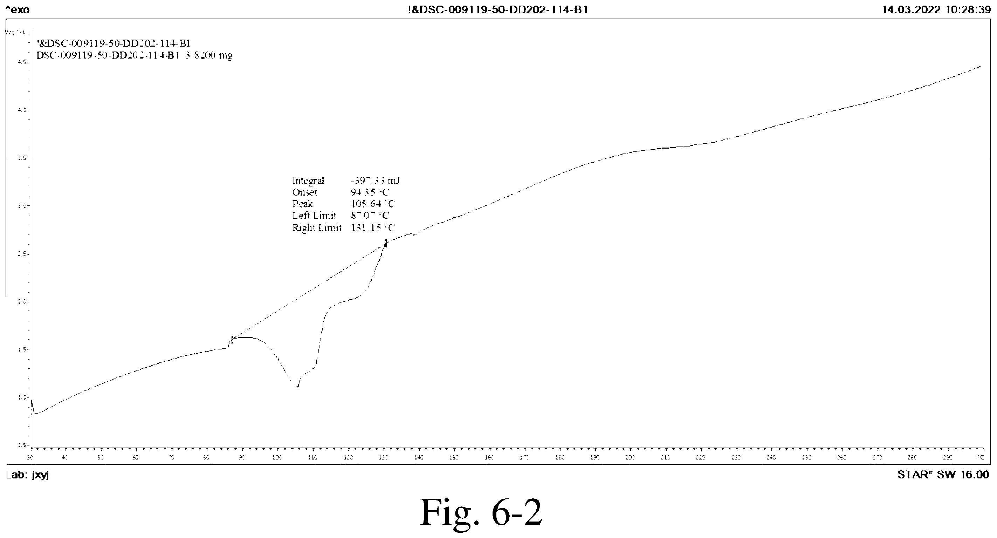

In some preferred embodiments, the DSC pattern of the Crystal Form E is substantially as shown in Figure 6-2.

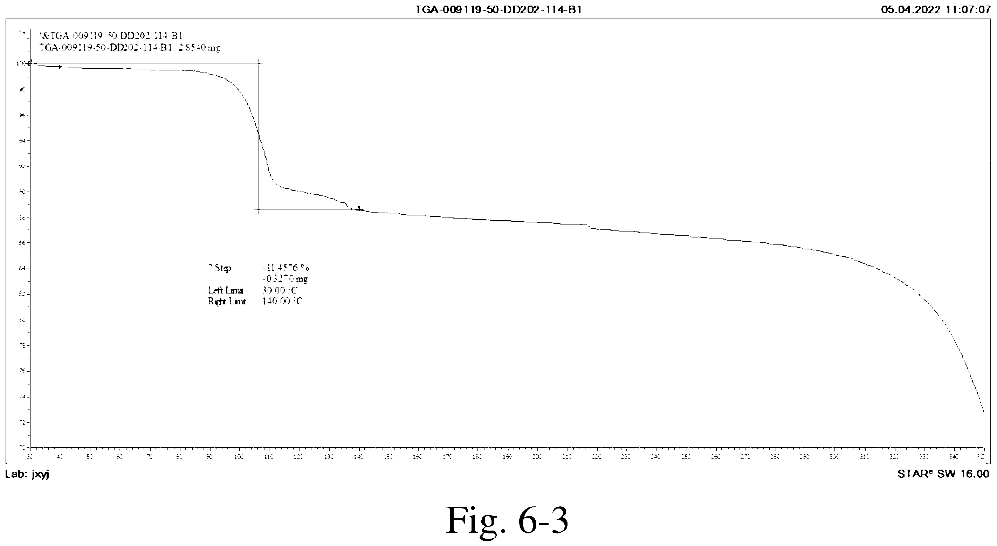

In some preferred embodiments, the TGA pattern of the Crystal Form E is substantially as shown in Figure 6-3.

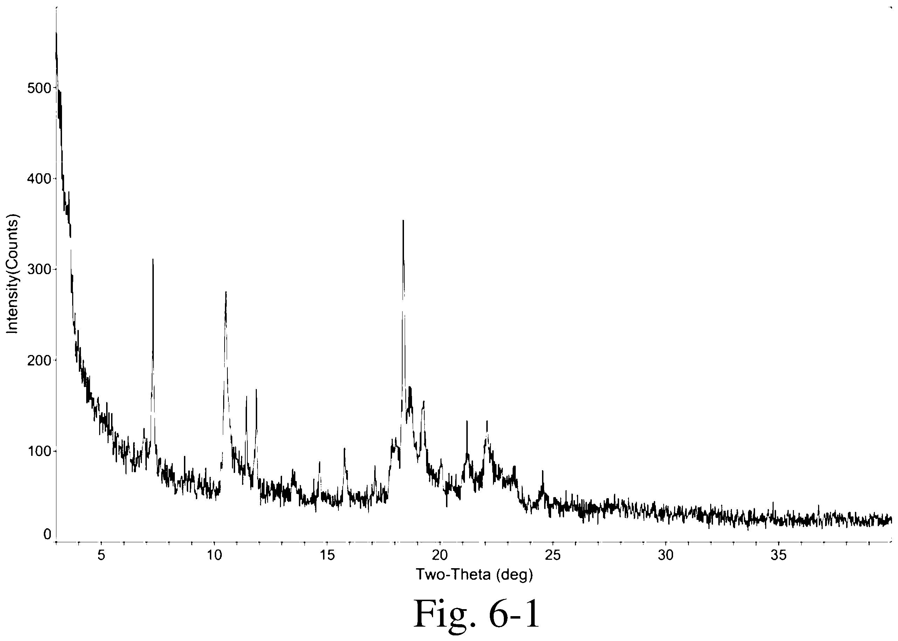

In some more preferred embodiments, the XRPD pattern of the Crystal Form E includes diffraction peaks at diffraction angles (2θ) that are substantially the same as those shown in Figure 6-1, and further more preferably, the XRPD pattern of F the Crystal Form E is as shown in Figure 6-1.

In some embodiments, the Crystal Form E is a solvate, more specifically a solvate with methyl tert-butyl ether (MTBE), wherein the stoichiometric ratio of the Compound I to the MTBE is preferably about 1:0.74.

In the DSC test, the Crystal Form E converts into an amorphous form when heated to 120 °C for 10 minutes.

In another aspect, the present invention also provides a method for preparing the Crystal Form E, comprising:
suspending the amorphous form of Compound I in MTBE and stirring the resulting suspension at a suitable temperature to obtain the Crystal Form E as a solid precipitate.

In some embodiments, the suitable temperature is, for example, a temperature of about 40-70 °C, such as a temperature of about 45-65 °C or about 50-60 °C, preferably about 50 °C.

In some embodiments, the stirring can be carried out for a suitable period of time, for example, about 1-2 days.

### vi. Crystal Form Ix

The present invention also provides Form Ix of Compound I, characterized in that the XRPD pattern of the Crystal Form Ix includes diffraction peaks at the following diffraction angles (2θ): about 3.70 ± 0.2°, 7.47 ± 0.2°, 11.24 ± 0.2°, 11.46 ± 0.2°, 15.02 ± 0.2°, 18.82 ± 0.2°, 19.68 ± 0.2°, 22.64 ± 0.2°, and 8.24 ± 0.2°.

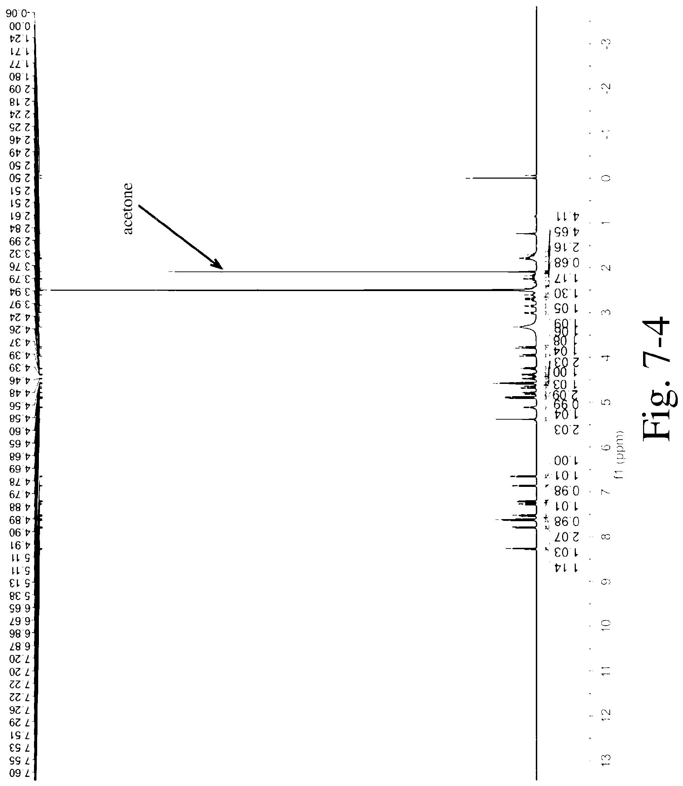

Alternatively or additionally, the Crystal Form Ix has any one, two, or all of the following characteristics:
(1) the DSC pattern of the Crystal Form Ix has one endothermic peak with a peak at about 113.2 °C ± 3.0 °C;
(2) the Crystal Form Ix has a weight loss of about 8.68% during the process of heating to about 130 °C ± 3 °C, as measured by TGA; and
(3) the ¹H NMR spectrum of the Crystal Form Ix is substantially as shown in Figure 7-4.

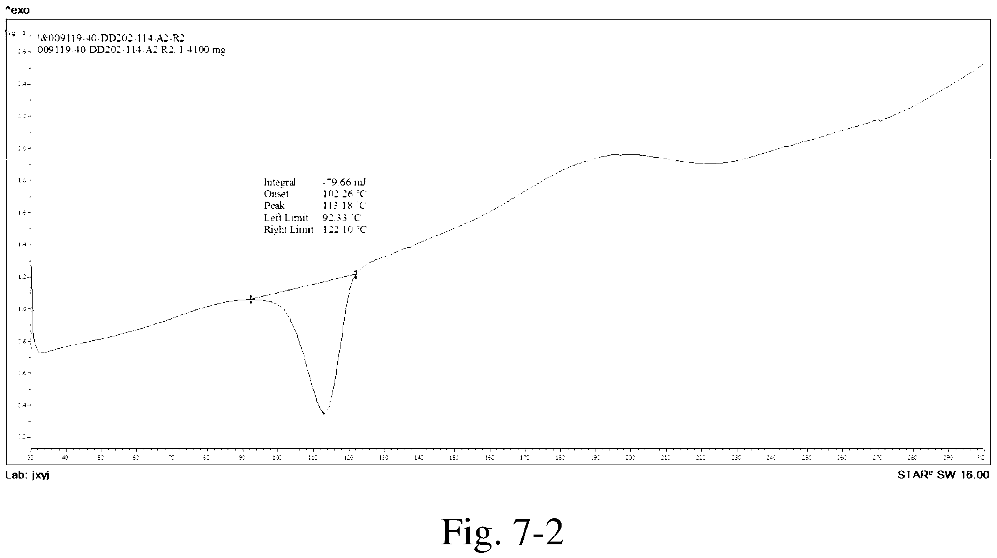

In some preferred embodiments, the DSC pattern of the Crystal Form Ix is substantially as shown in Figure 7-2.

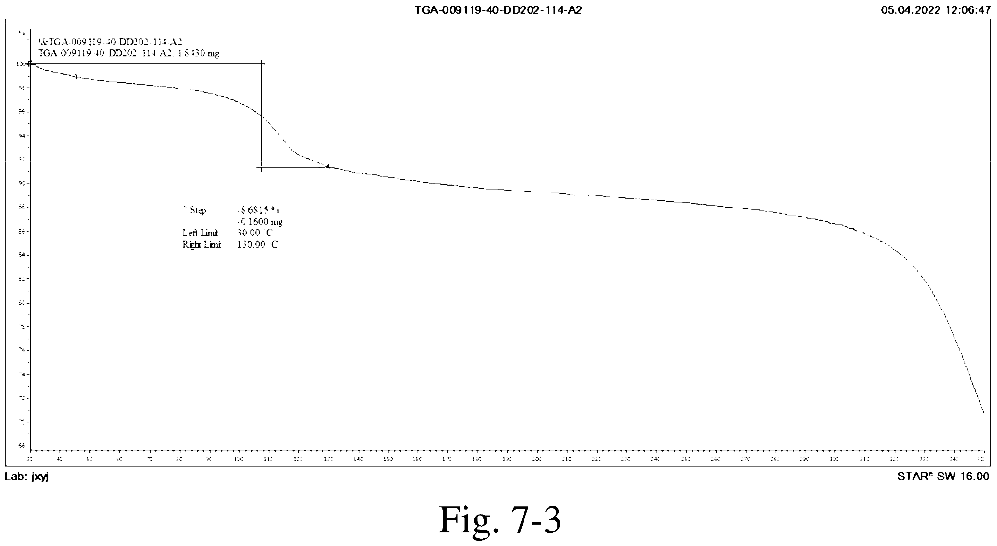

In some preferred embodiments, the TGA pattern of the Crystal Form Ix is substantially as shown in Figure 7-3.

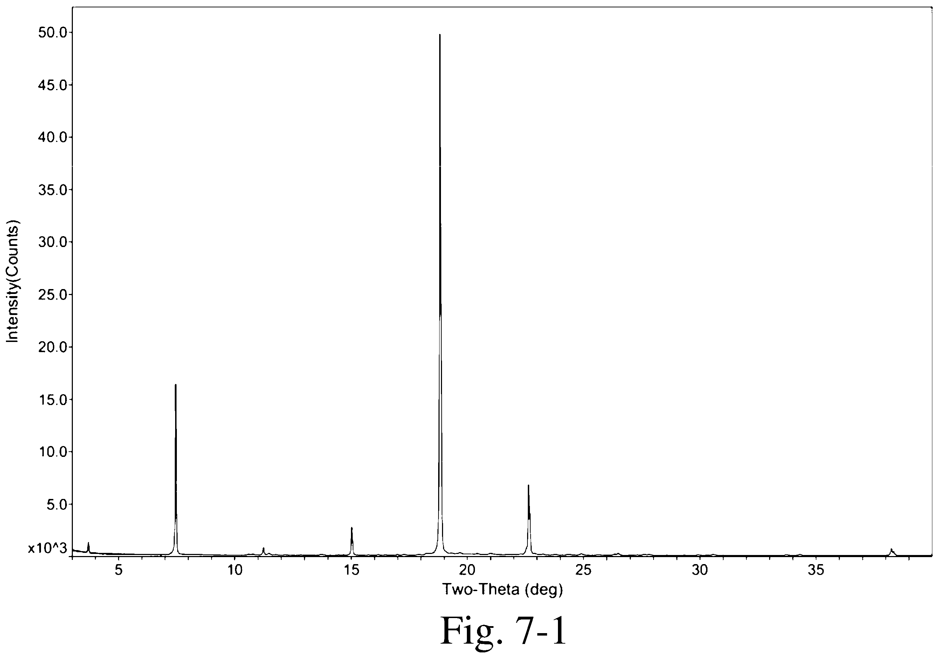

In some more preferred embodiments, the XRPD pattern of the Crystal Form Ix includes diffraction peaks at diffraction angles (2θ) that are substantially the same as those shown in Figure 7-1, and further more preferably, the XRPD pattern of the Crystal Form Ix is as shown in Figure 7-1.

In some embodiments, the Crystal Form Ix is a solvate, more specifically a solvate with acetone, wherein the stoichiometric ratio of the Compound I to the acetone is preferably about 1:0.78.

In the DSC test, the Crystal Form Ix converts into an amorphous form when heated to 120 °C for 10 minutes.

In another aspect, the present invention also provides a method for preparing the Crystal Form Ix of Compound I, comprising:
(1) providing a clear solution of Compound I in acetone; and
(2) allowing the solution to slowly evaporate at room temperature under conditions that allow for slow evaporation to obtain the Crystal Form Ix as a solid precipitate.

In some embodiments, the slow evaporation is carried out for a suitable period of time, for example, about 3 days.

### vii. Crystal Form J

The present invention also provides Crystal Form J of Compound I, characterized in that the XRPD pattern of the Crystal Form J includes diffraction peaks at the following diffraction angles (2θ): about 7.42 ± 0.2°, 18.10 ± 0.2°, 18.74 ± 0.2°, 19.03 ± 0.2°, and 22.13 ± 0.2°.

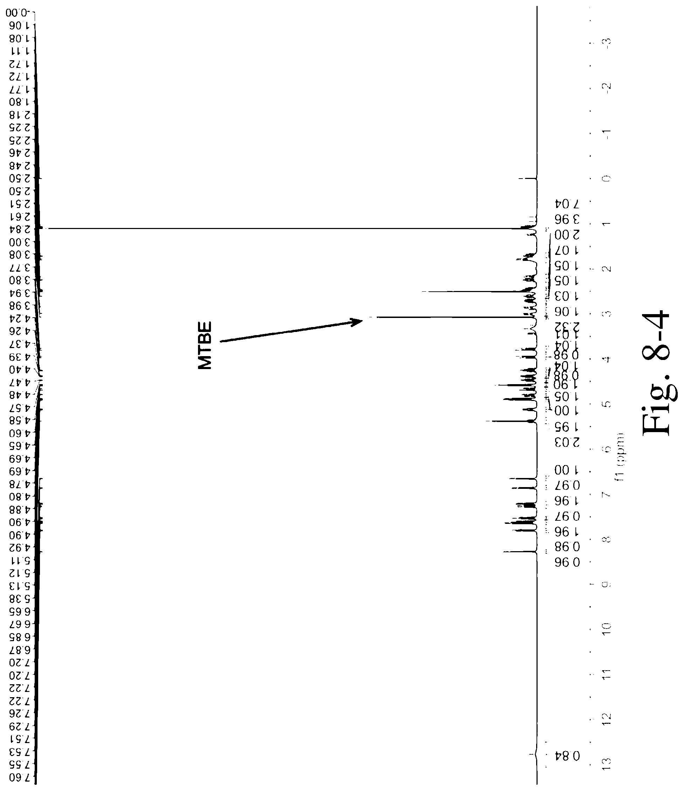

Alternatively or additionally, the Crystal Form J has any one, two, or all of the following characteristics:
(1) the DSC pattern of the Crystal Form J has one endothermic peak with a peak value at about 107.7°C ± 3.0°C;
(2) the Crystal Form J has a weight loss of about 10.35% during the process of heating to about 130°C ± 3°C, as measured by TGA; and
(3) the ¹H NMR spectrum of the Crystal Form J is substantially as shown in Figure 8-4.

In some preferred embodiments, the XRPD pattern of the Crystal Form J further includes diffraction peaks at any one, more, or all of the following diffraction angles (2θ): about 3.68 ± 0.2°, 10.58 ± 0.2°, 10.77 ± 0.2°, 11.01 ± 0.2°, 14.95 ± 0.2°, 22.56 ± 0.2°, 24.54 ± 0.2°, and 27.27 ± 0.2°.

In some preferred embodiments, the XRPD pattern of the Crystal Form J further includes diffraction peaks at any one, more, or all of the following diffraction angles (2θ): about 11.67 ± 0.2°, 13.62 ± 0.2°, 16.32 ± 0.2°, 17.59 ± 0.2°, 20.53 ± 0.2°, 25.09 ± 0.2°, and 26.05 ± 0.2°.

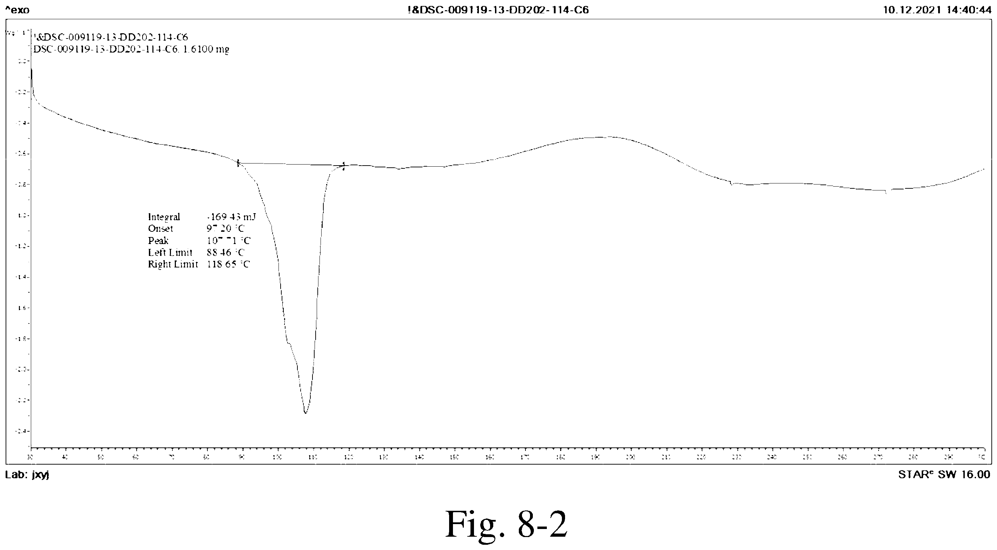

In some preferred embodiments, the DSC pattern of the Crystal Form J is substantially as shown in Figure 8-2.

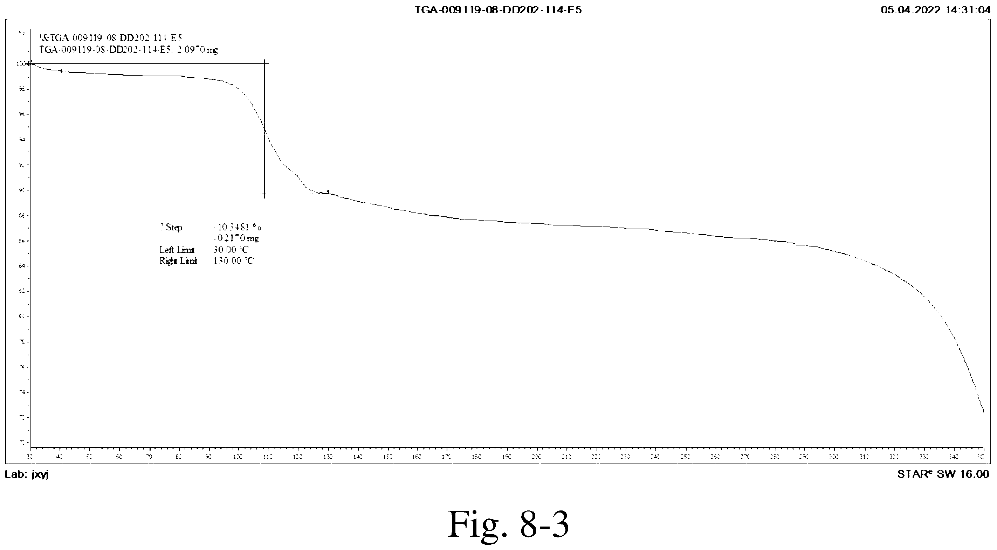

In some preferred embodiments, the TGA pattern of the Crystal Form J is substantially as shown in Figure 8-3.

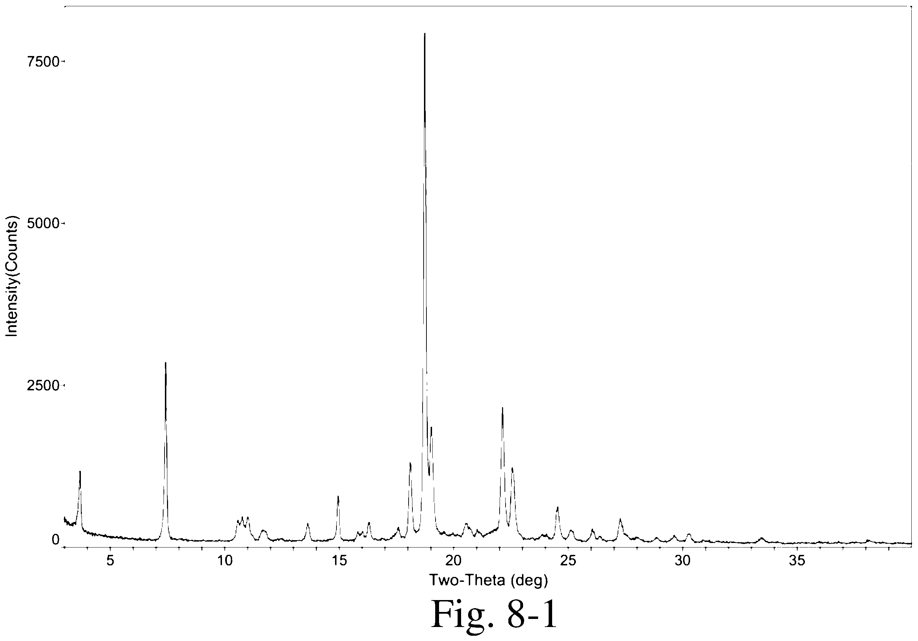

In some more preferred embodiments, the XRPD pattern of the Crystal Form J includes diffraction peaks at diffraction angles (2θ) that are substantially the same as those shown in Figure 8-1, and further more preferably, the XRPD pattern of the Crystal Form J is as shown in Figure 8-1.

In some embodiments, the Crystal Form J is a solvate, more specifically a solvate with MTBE, wherein the stoichiometric ratio of the Compound I to the MTBE is preferably about 1:0.77.

In the DSC test, the Crystal Form J transforms into an amorphous form when heated to 120°C for 10 minutes.

In another aspect, the present invention also provides a method for preparing the Crystal Form J, comprising:
(1) providing a clear solution of Compound I in a mixture of EtOH:MTBE (for example, at about 1:3, v:v) at an elevated temperature; and
(2) allowing the solution to cool naturally to room temperature to obtain the Crystal Form J as a solid precipitate.

In some embodiments, the elevated temperature is a temperature of about 40-60°C, such as a temperature of about 45-55°C or about 50-55°C, preferably about 50°C.

In some embodiments, the method comprises stirring a suspension of Compound I in the mixture of EtOH:MTBE, and then heating the suspension to the elevated temperature so that Compound I dissolves to form the solution.

### viii. Crystal Form K

The present invention also provides Crystal Form K of Compound I, characterized in that the XRPD pattern of the Crystal Form K includes diffraction peaks at the following diffraction angles (2θ): about 3.70 ± 0.2°, 7.44 ± 0.2°, 18.74 ± 0.2°, 19.40 ± 0.2°, and 22.38 ± 0.2°.

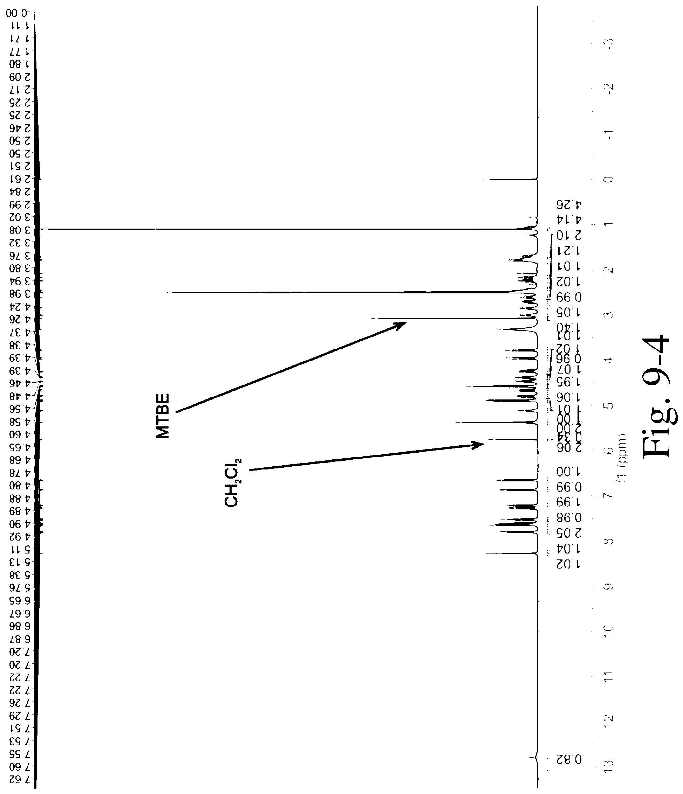

Alternatively or additionally, the Crystal Form K has any one, two, or all of the following characteristics:
(1) the DSC pattern of the Crystal Form K has one endothermic peak with a peak value at about 103.8°C ± 3.0 °C;
(2) the Crystal Form K has a weight loss of about 8.47% during the process of heating to about 110°C ± 3 °C, as measured by TGA; and
(3) the ¹H NMR spectrum of the Crystal Form K is substantially as shown in Figure 9-4.

In some preferred embodiments, the XRPD pattern of the Crystal Form K further includes diffraction peaks at any one, more, or all of the following diffraction angles (2θ): about 11.20 ± 0.2°, 11.81 ± 0.2°, 14.94 ± 0.2°, 24.73 ± 0.2°, 26.15 ± 0.2°, and 27.45 ± 0.2°.

In some preferred embodiments, the XRPD pattern of the Crystal Form K further includes diffraction peaks at any one, more, or all of the following diffraction angles (2θ): about 13.76 ± 0.2°, 15.82 ± 0.2°, 16.44 ± 0.2°, 17.65 ± 0.2°, 21.14 ± 0.2°, 23.92 ± 0.2°, and 25.29 ± 0.2°.

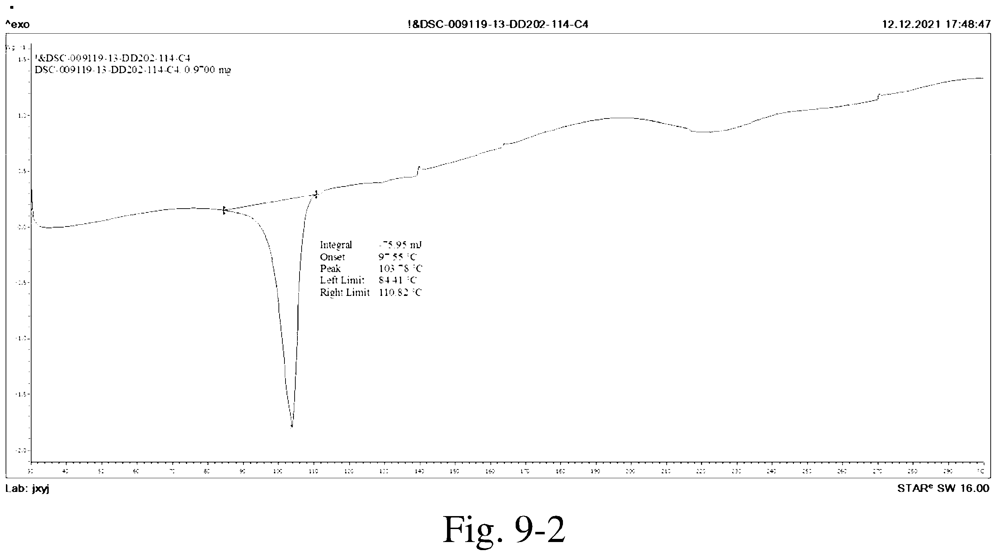

In some preferred embodiments, the DSC pattern of the Crystal Form K is substantially as shown in Figure 9-2.

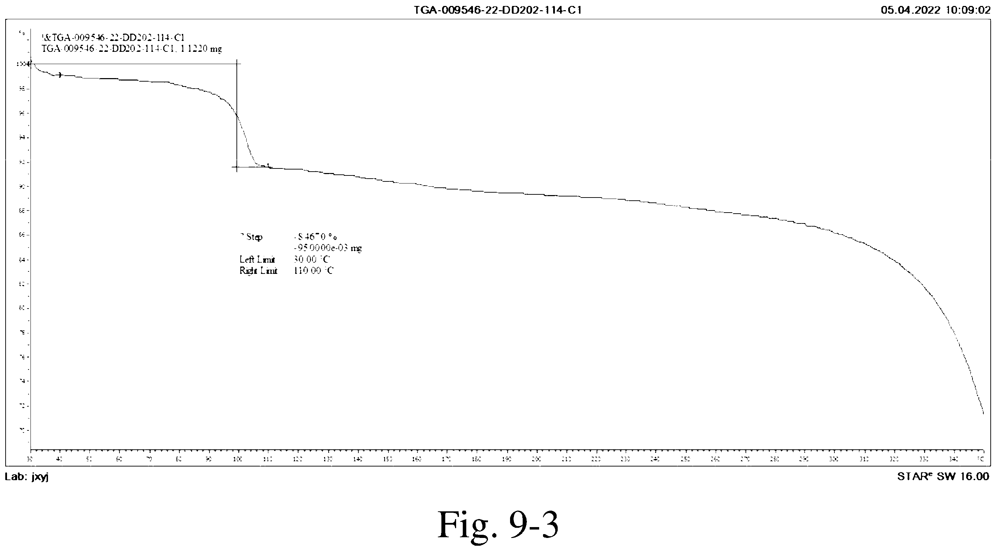

In some preferred embodiments, the TGA pattern of the Crystal Form K is substantially as shown in Figure 9-3.

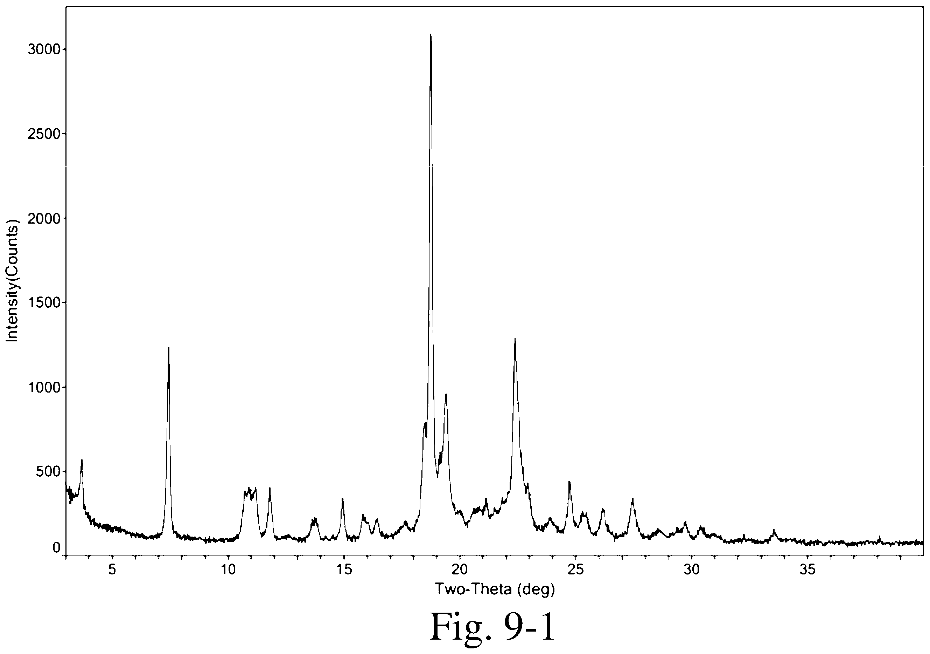

In some more preferred embodiments, the XRPD pattern of the Crystal Form K includes diffraction peaks at diffraction angles (2θ) that are substantially the same as those shown in Figure 9-1, and further more preferably, the XRPD pattern of the Crystal Form K is as shown in Figure 9-1.

In some embodiments, the Crystal Form K is a solvate, more specifically a solvate with MTBE, wherein the stoichiometric ratio of the Compound I to the MTBE is preferably about 1:0.47.

In the DSC test, the Crystal Form K transforms into an amorphous form when heated to 110 °C for 10 minutes.

In another aspect, the present invention also provides a method for preparing the Crystal Form K, comprising:
(1) providing a clear solution of Compound I in a mixture of CH₂Cl₂:MTBE (for example, at about 1:3, v:v) at an elevated temperature; and
(2) allowing the solution to cool naturally to room temperature to obtain the Crystal Form K as a solid precipitate.

In some embodiments, the elevated temperature is a temperature of about 40-60 °C, such as a temperature of about 45-55 °C or about 50-55 °C, preferably about 50 °C.

In some embodiments, the method comprises stirring a suspension of Compound I in the mixture of CH₂Cl₂:MTBE, and then heating the suspension to the elevated temperature so that Compound I dissolves to form the solution.

### ix. Crystal Form L

The present invention also provides Crystal Form L of Compound I, characterized in that the XRPD pattern of the Crystal Form L includes diffraction peaks at the following diffraction angles (2θ): about 7.41 ± 0.2°, 11.18 ± 0.2°, 18.72 ± 0.2°, 19.75 ± 0.2°, and 22.42 ± 0.2°.

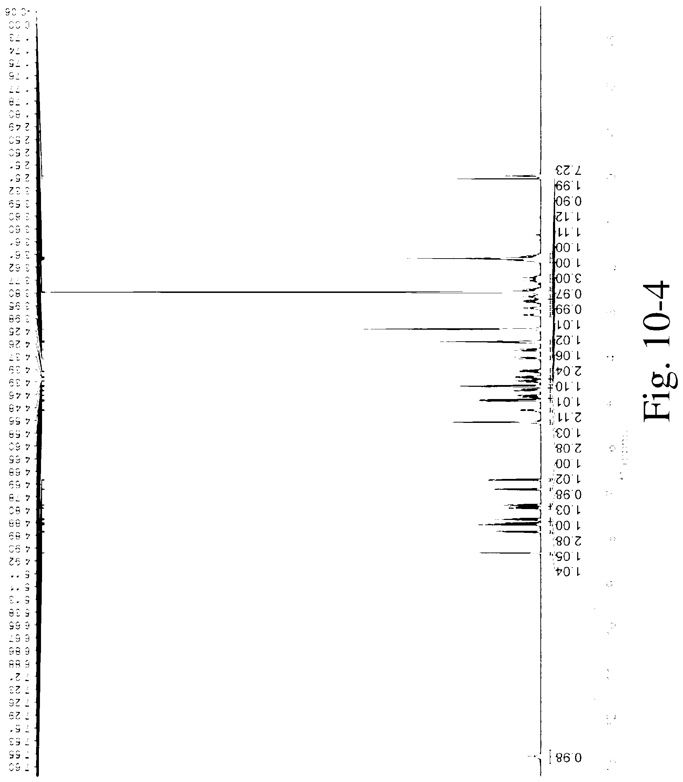

Alternatively or additionally, the Crystal Form L has any one, two, or all of the following characteristics:
(1) the DSC pattern of the Crystal Form L has one endothermic peak with a peak value at about 98.1°C ± 3.0 °C;
(2) the Crystal Form L has a weight loss of about 7.62% during the process of heating to about 110°C ± 3 °C, as measured by TGA; and
(3) the ¹H NMR spectrum of the Crystal Form L is substantially as shown in Figure 10-4.

In some preferred embodiments, the XRPD pattern of the Crystal Form L further includes diffraction peaks at any one, more, or all of the following diffraction angles (2θ): about 10.97 ± 0.2°, 11.76 ± 0.2°, 24.73 ± 0.2°, 26.34 ± 0.2°, and 27.32 ± 0.2°.

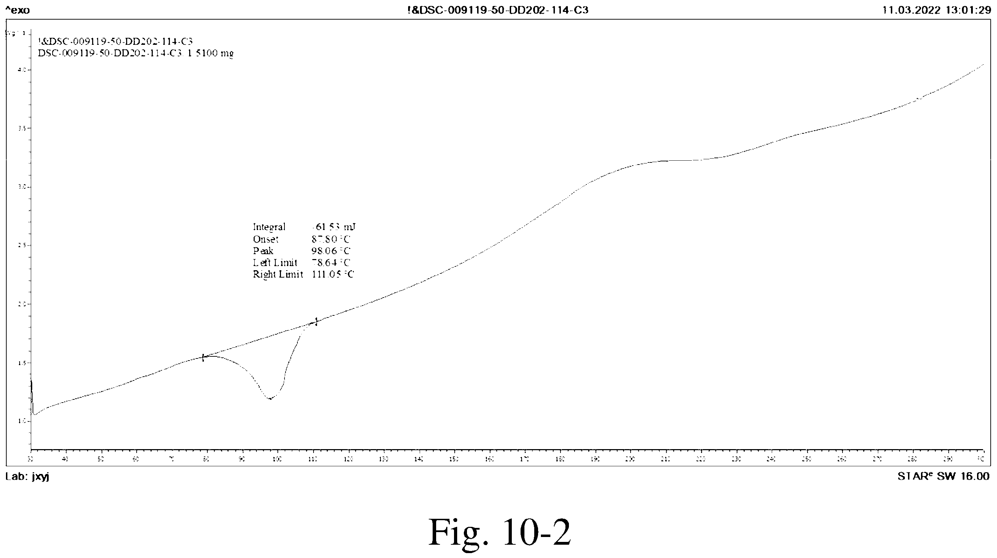

In some preferred embodiments, the DSC pattern of the Crystal Form L is substantially as shown in Figure 10-2.

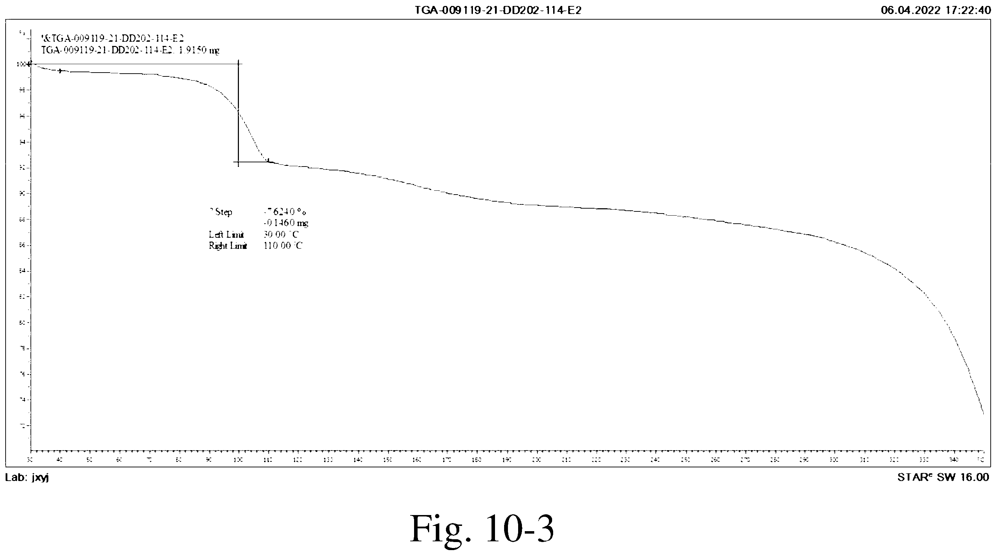

In some preferred embodiments, the TGA pattern of the Crystal Form L is substantially as shown in Figure 10-3.

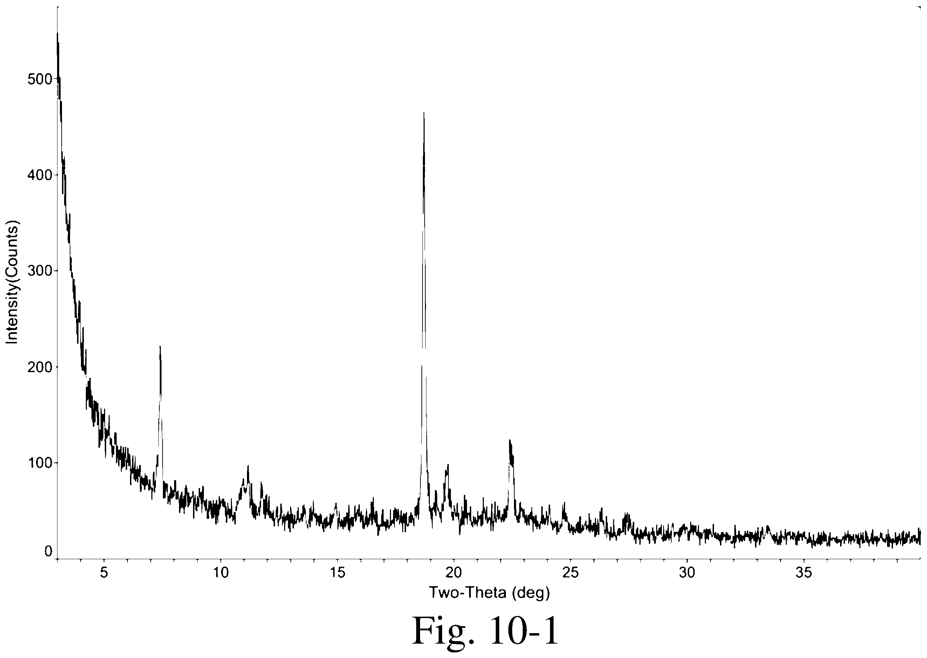

In some more preferred embodiments, the XRPD pattern of the Crystal Form L includes diffraction peaks at diffraction angles (2θ) that are substantially the same as those shown in Figure 10-1, and further more preferably, the XRPD pattern of the Crystal Form L is as shown in Figure 10-1.

In some embodiments, the Crystal Form L is a solvate, more specifically a solvate with THF, wherein the stoichiometric ratio of the Compound I to the THF is preferably about 1:0.75.

In the DSC test, the Crystal Form L transforms into an amorphous form when heated to 120 °C for 10 minutes.

In another aspect, the present invention also provides a method for preparing the Crystal Form L, comprising:
(1) providing a clear solution of Compound I in a mixture of THF:H₂O (for example, at about 1:2, v:v) at an elevated temperature; and
(2) allowing the solution to cool naturally to room temperature to obtain the Crystal Form L as a solid precipitate.

In some embodiments, the elevated temperature is a temperature of about 40-60 °C, such as a temperature of about 45-55 °C or about 50-55 °C, preferably about 50 °C.

In some embodiments, the method comprises stirring a suspension of Compound I in the mixture of THF:H₂O, and then heating the suspension to the elevated temperature so that Compound I dissolves to form the solution.

### x. Crystal Form M

The present invention also provides Crystal Form M of Compound I, characterized in that the XRPD pattern of the Crystal Form M includes diffraction peaks at the following diffraction angles (2θ): about 3.51 ± 0.2°, 7.11 ± 0.2°, 14.28 ± 0.2°, 17.93 ± 0.2°, 21.58 ± 0.2°, and 25.25 ± 0.2°.

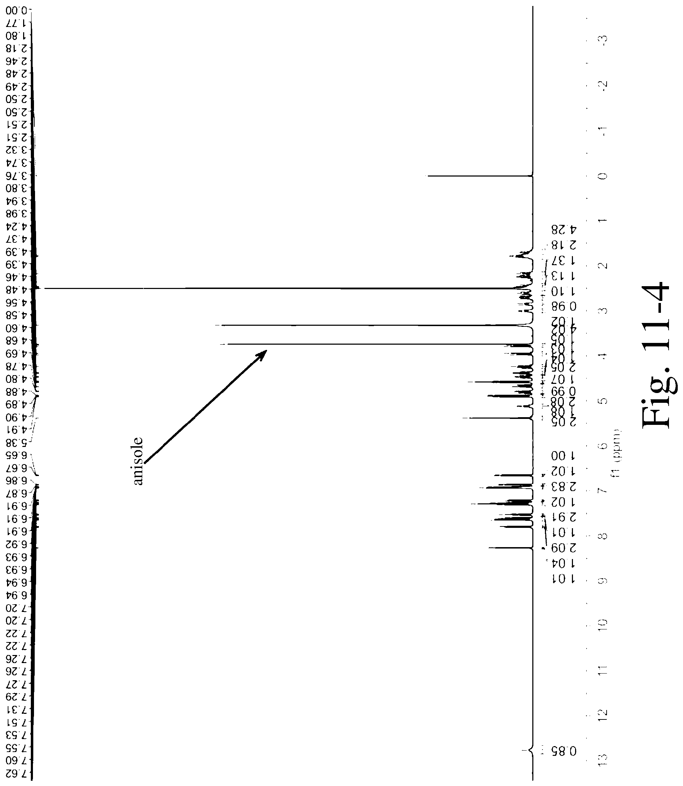

**Alternatively** or additionally, the Crystal Form M has any one, two, or all of the following characteristics:
(1) the DSC pattern of the Crystal Form M has two endothermic peaks with peak values at about 95.8°C ± 3.0 °C and about 109.2°C ± 3.0 °C, respectively;
(2) the Crystal Form M has a weight loss of about 15.55% during the process of heating to about 230°C ± 3 °C, as measured by TGA; and
(3) the ¹H NMR spectrum of the Crystal Form M is substantially as shown in Figure 11-4.

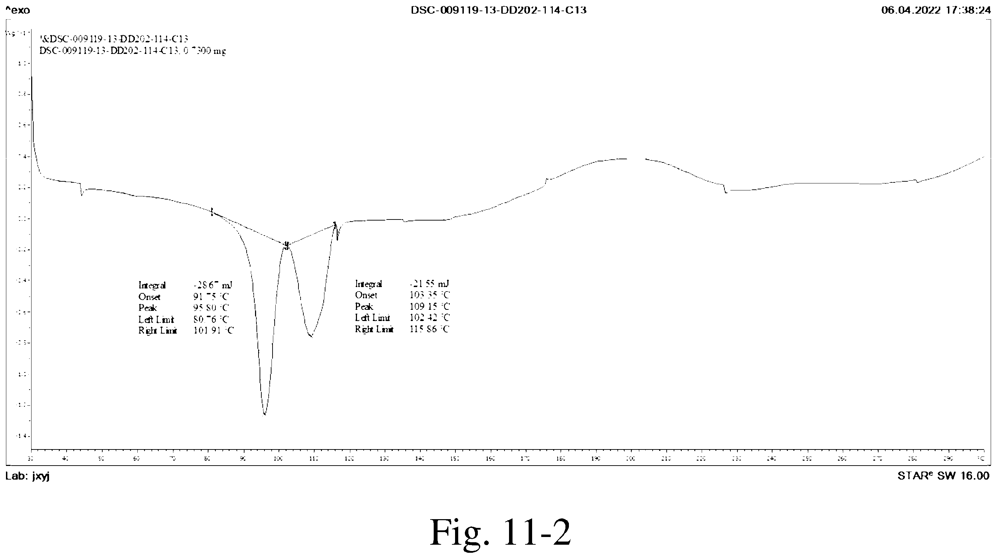

In some preferred embodiments, the DSC pattern of the Crystal Form M is substantially as shown in Figure 11-2.

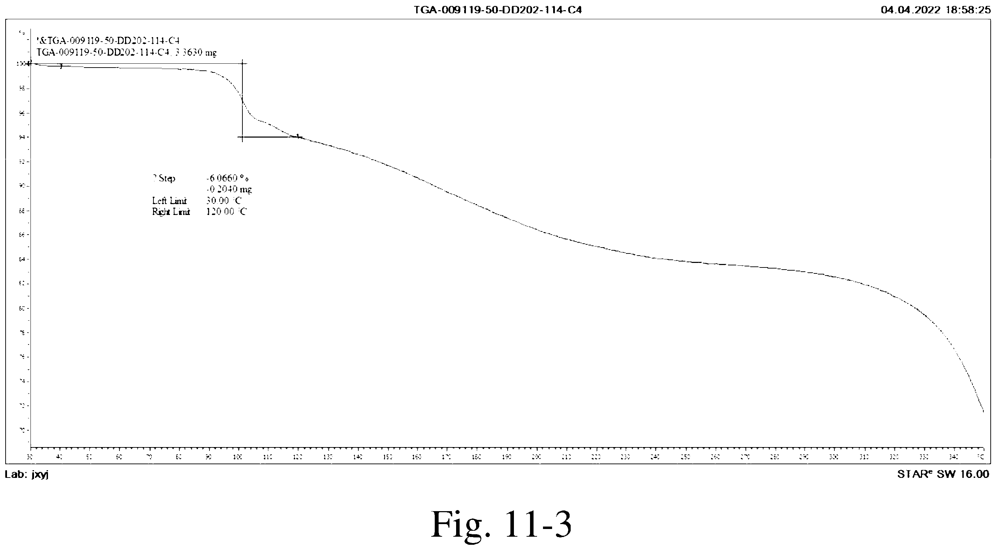

In some preferred embodiments, the TGA pattern of the Crystal Form M is substantially as shown in Figure 11-3.

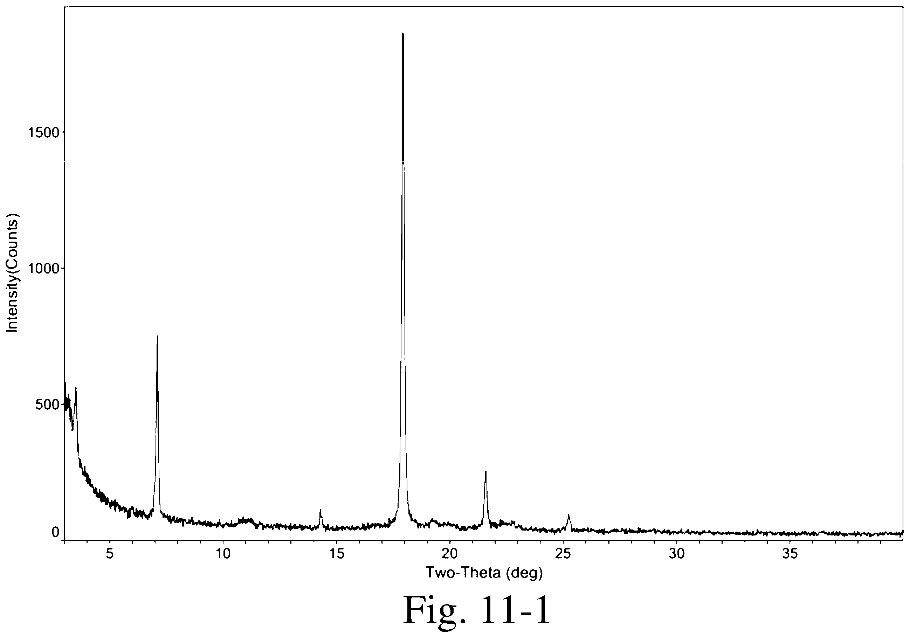

In some more preferred embodiments, the XRPD pattern of the Crystal Form M includes diffraction peaks at diffraction angles (2θ) that are substantially the same as those shown in Figure 11-1, and further more preferably, the XRPD pattern of the Crystal Form M is as shown in Figure 11-1.

In some embodiments, the Crystal Form M is a solvate, more specifically a solvate with anisole, wherein the stoichiometric ratio of the Compound I to the anisole is preferably about 1:1.

In the DSC test, the Crystal Form M transforms into an amorphous form when heated to 105 °C for 10 minutes.

In another aspect, the present invention also provides a method for preparing the Crystal Form M, comprising:
(1) providing a clear solution of Compound I in a mixture of anisole:HEP (for example, at about 9:1, v:v) at an elevated temperature; and
(2) allowing the solution to cool naturally to room temperature to obtain the Crystal Form M as a solid precipitate.

In some embodiments, the elevated temperature is a temperature of about 40-60 °C, such as a temperature of about 45-55 °C or about 50-55 °C, preferably about 50 °C.

In some embodiments, the method comprises stirring a suspension of Compound I in the mixture of anisole:HEP, and then heating the suspension to the elevated temperature so that Compound I dissolves to form the solution.

### xi. Crystal Form N

The present invention further provides Crystal Form N of Compound **I,** characterized in that the XRPD pattern of the Crystal Form N includes diffraction peaks at the following diffraction angles (2θ): about 3.57 ± 0.2°, 7.23 ± 0.2°, 14.57 ± 0.2°, 18.26 ± 0.2°, and 21.96 ± 0.2°.

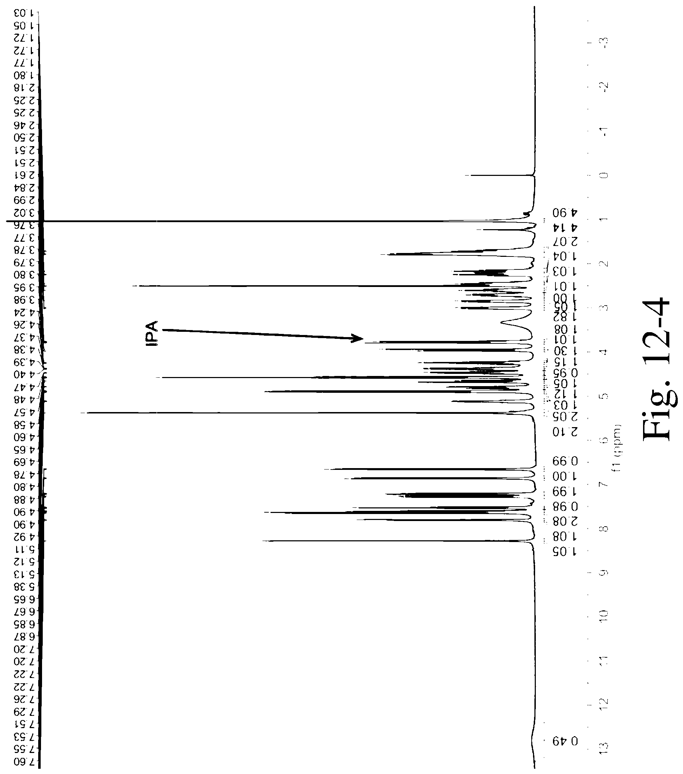

Alternatively or additionally, the Crystal Form N has any one, two, or all of the following characteristics:
(1) the DSC pattern of the Crystal Form N has one endothermic peak with a peak value at about 106.8°C ± 3.0 °C;
(2) the Crystal Form N has a weight loss of about 11.80% during the process of heating to about 180°C ± 3 °C, as measured by TGA; and
(3) the ¹H NMR spectrum of the Crystal Form N is substantially as shown in Figure 12-4.

In some preferred embodiments, the XRPD pattern of the Crystal Form N further includes diffraction peaks at any one, more, or all of the following diffraction angles (2θ): about 10.91 ± 0.2°, 22.95 ± 0.2°, 23.88 ± 0.2°, and 37.08 ± 0.2°.

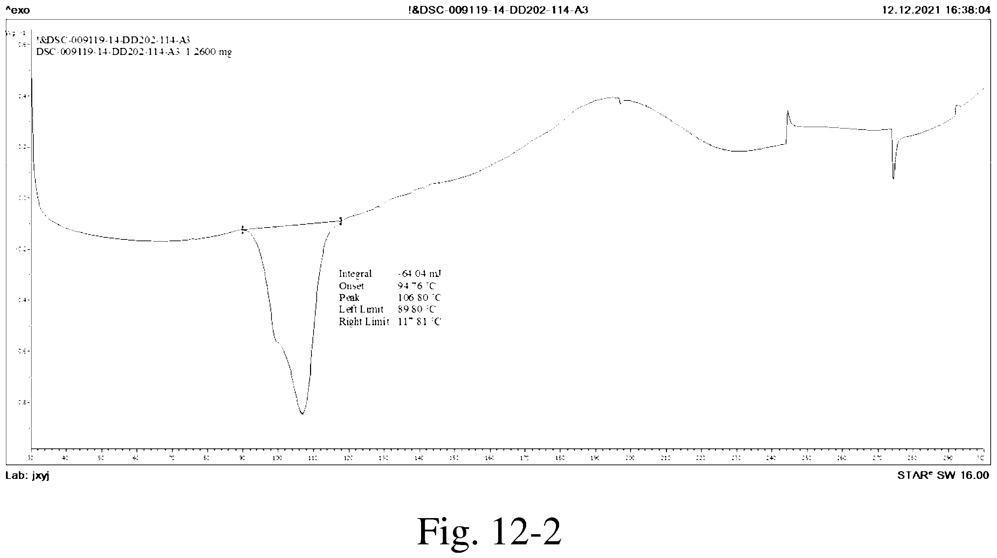

In some preferred embodiments, the DSC pattern of the Crystal Form N is substantially as shown in Figure 12-2.

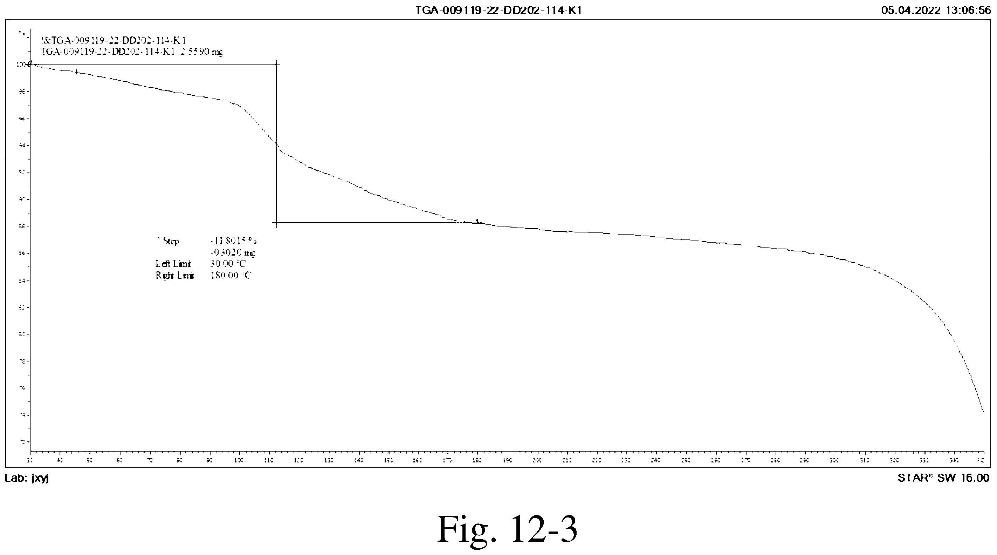

In some preferred embodiments, the TGA pattern of the Crystal Form N is substantially as shown in Figure 12-3.

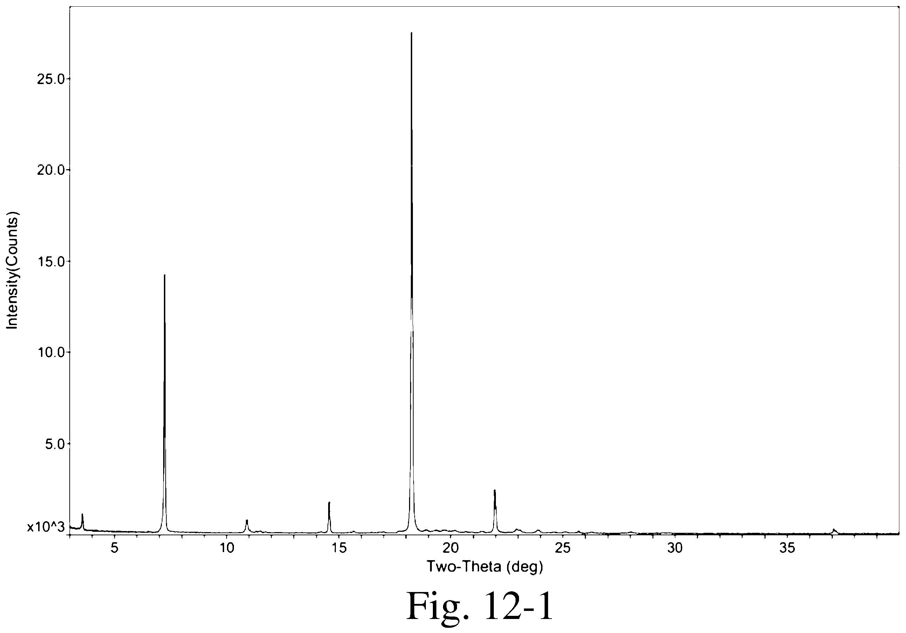

In some more preferred embodiments, the XRPD pattern of the Crystal Form N includes diffraction peaks at diffraction angles (2θ) that are substantially the same as those shown in Figure 12-1, and further more preferably, the XRPD pattern of the Crystal Form N is as shown in Figure 12-1.

In some embodiments, the Crystal Form N is a solvate, more specifically a solvate with isopropyl alcohol (IPA), wherein the stoichiometric ratio of the Compound I to the IPA is preferably about 1: 0.82.

In the DSC test, the Crystal Form N transforms into an amorphous form when heated to 120 °C for 10 minutes.

In another aspect, the present invention also provides a method for preparing the Crystal Form N of Compound I, comprising:
(1) providing a solution of Compound I in a mixture of IPA:H₂O (e.g., at about 4:1, v:v); and
(2) allowing the solution to slowly evaporate at room temperature under conditions that allow for slow evaporation to obtain the Crystal Form N as a solid precipitate.

In some embodiments, the slow evaporation is carried out for a suitable period of time, such as about 6-10 days or about 8-9 days.

### xii. Crystal Form O

The present invention further provides Crystal Form O of Compound I, characterized in that the XRPD pattern of the Crystal Form O includes diffraction peaks at the following diffraction angles (2θ): about 7.54 ± 0.2°, 10.95 ± 0.2°, 18.88 ± 0.2°, 19.79 ± 0.2°, and 22.62 ± 0.2°.

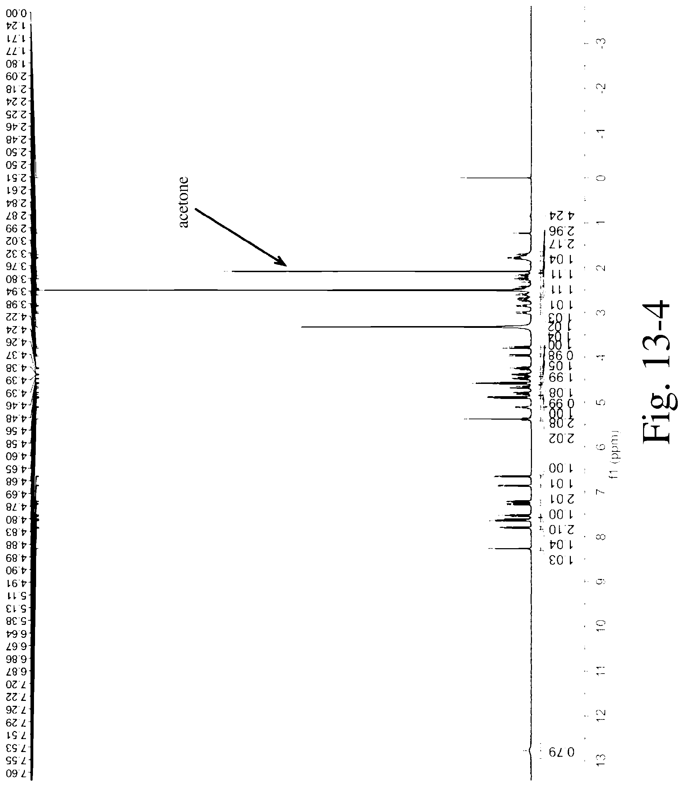

Alternatively or additionally, the Crystal Form O has any one, two, or all of the following characteristics:
(1) the DSC pattern of the Crystal Form O has one endothermic peak with a peak value at about 95.5°C ± 3.0 °C;
(2) the Crystal Form O has a weight loss of about 5.38% during the process of heating to about 110°C ± 3 °C, as measured by TGA.
(3) the ¹H NMR spectrum of the Crystal Form O is substantially as shown in Figure 13-4.

In some preferred embodiments, the XRPD pattern of the Crystal Form O further includes diffraction peaks at any one, more, or all of the following diffraction angles (2θ): about 11.44 ± 0.2°, 11.88 ± 0.2°, 13.75 ± 0.2°, and 21.19 ± 0.2°.

In some preferred embodiments, the XRPD pattern of the Crystal Form O further includes diffraction peaks at any one, more, or all of the following diffraction angles (2θ): about 15.05 ± 0.2°, 15.87 ± 0.2°, 16.17 ± 0.2°, 24.95 ± 0.2°, 26.44 ± 0.2°, and 27.66 ± 0.2°.

In some preferred embodiments, the XRPD pattern of the Crystal Form O further includes diffraction peaks at any one, more, or all of the following diffraction angles (2θ): about 17.45 ± 0.2°, 21.86 ± 0.2°, 23.85 ± 0.2°, and 25.72 ± 0.2°.

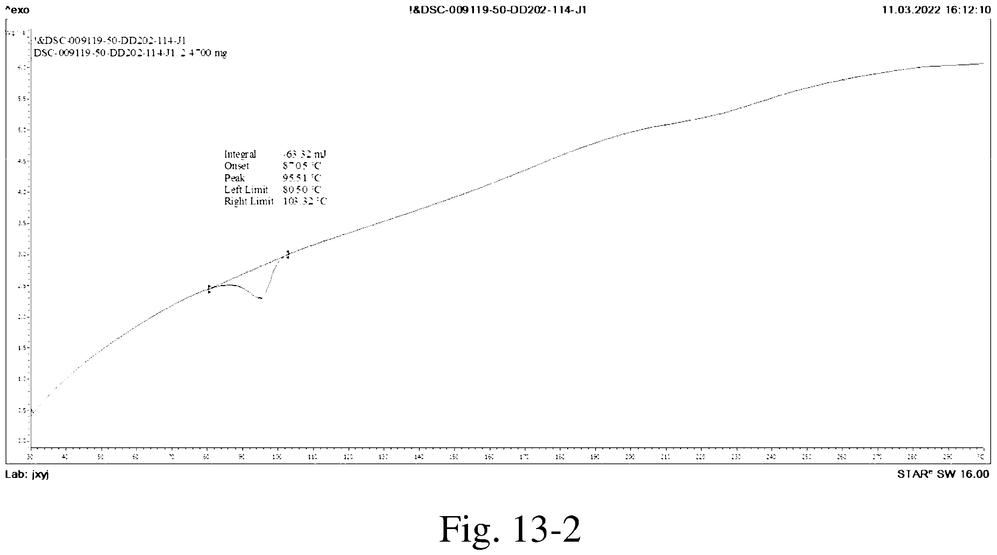

In some preferred embodiments, the DSC pattern of the Crystal Form O is substantially as shown in Figure 13-2.

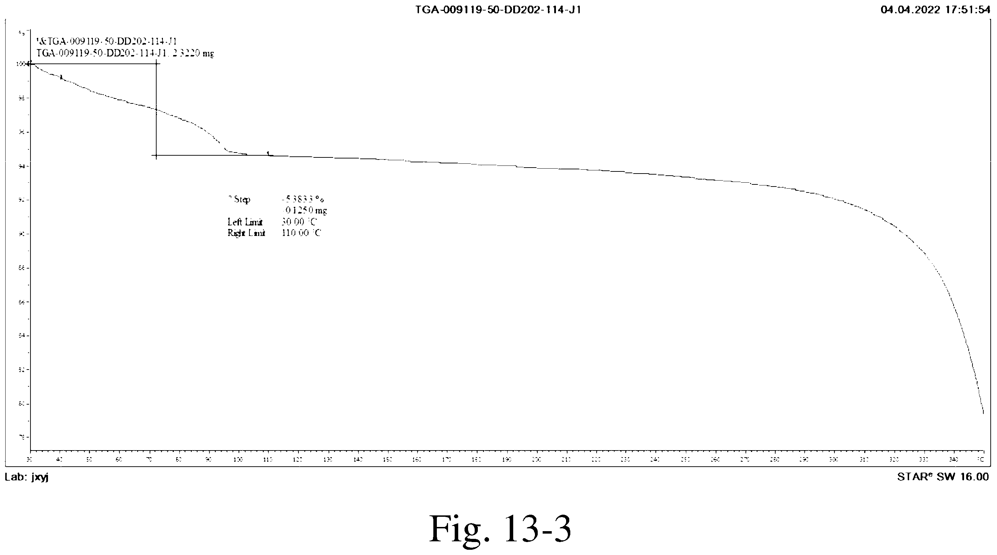

In some preferred embodiments, the TGA pattern of the Crystal Form O is substantially as shown in Figure 13-3.

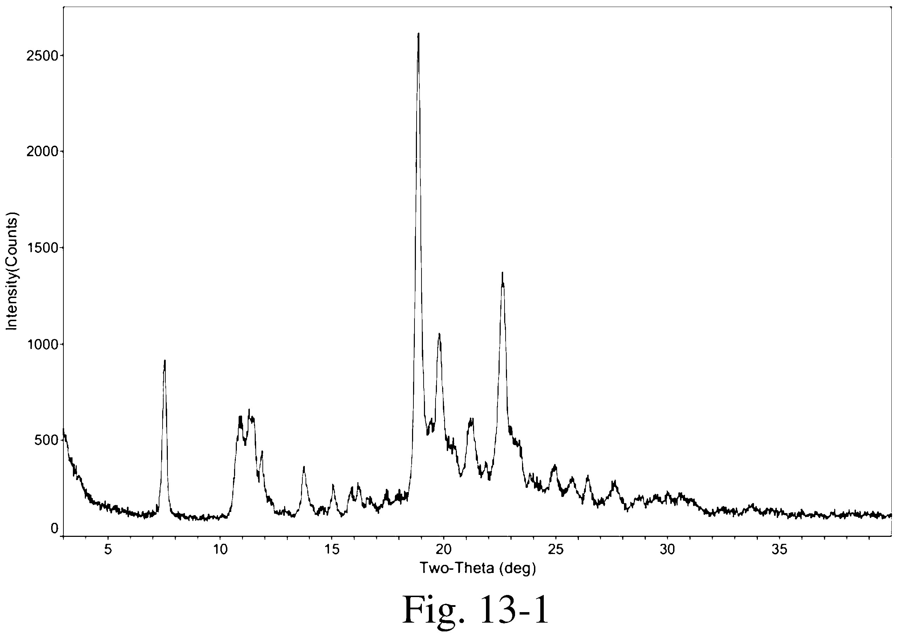

In some more preferred embodiments, the XRPD pattern of the Crystal Form O includes diffraction peaks at diffraction angles (2θ) that are substantially the same as those shown in Figure 13-1, and further more preferably, the XRPD pattern of the Crystal Form O is as shown in Figure 13-1.

In some embodiments, the Crystal Form O is a solvate, more specifically a solvate with acetone, wherein the stoichiometric ratio of the Compound I to the acetone is preferably about 1:0.5.

In the DSC test, the Crystal Form O transforms into an amorphous form when heated to 120 °C for 10 minutes.

In another aspect, the present invention also provides a method for preparing the Crystal Form O of Compound I, comprising:
contacting the amorphous form of Compound I with acetone vapor in a sealed container for a suitable period of time to obtain the Crystal Form O.

In some embodiments, the suitable period of time can be, for example, about 9-10 days.

### xiii. Other Crystal Forms

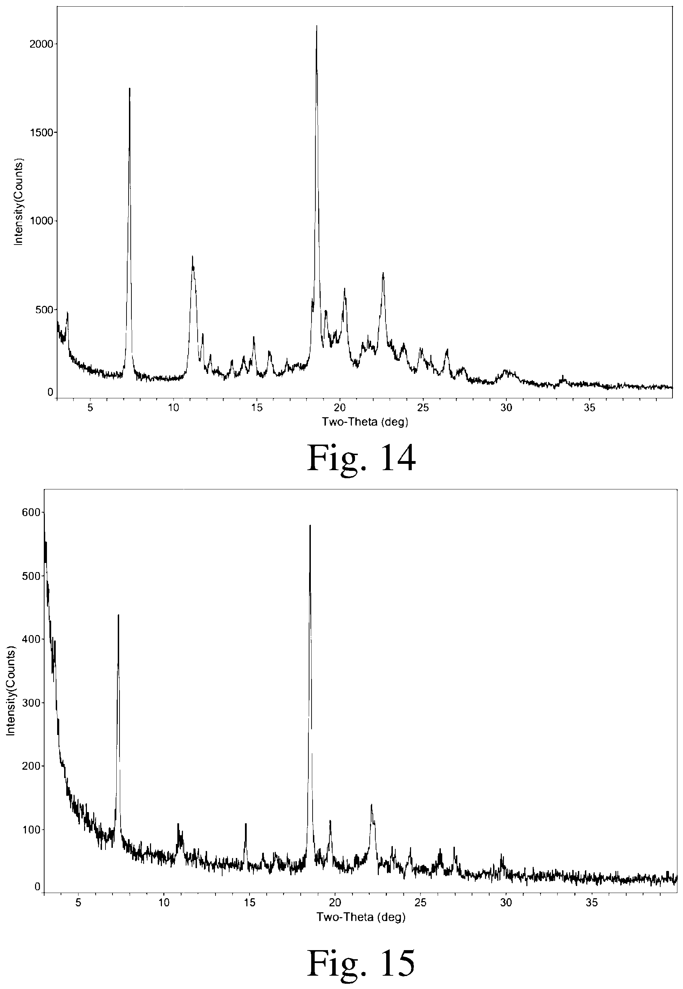

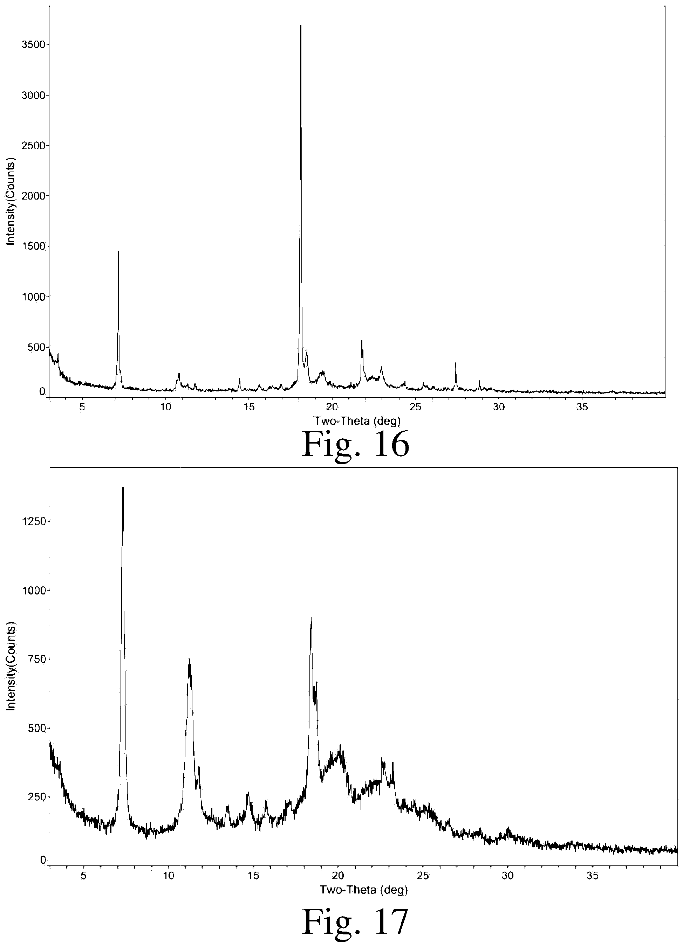

The present invention also provides metastable crystal forms of Compound I, including:
(1) Crystal Form B, having an XRPD pattern substantially as shown in Figure 14, which transforms into an amorphous form after drying at about 50°C for 3 hours;
(2) Crystal Form H, having an XRPD pattern substantially as shown in Figure 15, which transforms into a weakly crystalline state after drying at about 50°C for 2 hours;
(3) Crystal Form F, having an XRPD pattern substantially as shown in Figure 16, which transforms into Crystal Form D after being stored under sealed conditions at room temperature for 21 days;
(4) Crystal Form P, having an XRPD pattern substantially as shown in Figure 17, which transforms into an amorphous form after drying at about 50°C for 3.5 hours.

### II. Pharmaceutical Compositions

In another aspect, the present invention provides a pharmaceutical composition comprising a crystalline form of Compound I of the present invention, wherein the crystalline form of Compound I is selected from Crystal Form A, Crystal Form B, Crystal Form C, Crystal Form D, Crystal Form E, Crystal Form F, Crystal Form G, Crystal Form H, Crystal Form Ix, Crystal Form J, Crystal Form K, Crystal Form L, Crystal Form M, Crystal Form N, Crystal Form O, and Crystal Form P as described above. Preferably, the crystalline form of Compound I is selected from Crystal Form A, Crystal Form C, Crystal Form D, Crystal Form E, Crystal Form G, Crystal Form Ix, Crystal Form J, Crystal Form K, Crystal Form L, Crystal Form M, Crystal Form N, and Crystal Form O. More preferably, the crystalline form of Compound I is the Crystal Form A or Crystal Form G, and even more preferably the Crystal Form G.

The present invention also provides the pharmaceutical composition as described above, which further comprises one, two, or more of other therapeutically active ingredients.

The pharmaceutical composition of the present invention can also comprise a pharmaceutically acceptable carrier.

In the present invention, a "pharmaceutically acceptable carrier" refers to a diluent, adjuvant, excipient, or vehicle with which a therapeutic agent is administered, and which is, within the scope of sound medical judgment, suitable for contact with the tissues of humans and/or other animals without excessive toxicity, irritation, allergic response, or other problems or complications commensurate with a reasonable benefit/risk ratio.

Pharmaceutically acceptable carriers that can be used in the pharmaceutical composition of the present invention include, but are not limited to, sterile liquids, such as water and oils, including those of petroleum, animal, vegetable, or synthetic origin, such as peanut oil, soybean oil, mineral oil, sesame oil, etc. When the pharmaceutical composition is administered intravenously, water is an exemplary carrier. Saline solutions and aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, skim milk powder, glycerol, propylene glycol, water, ethanol, etc. The composition may also, if desired, contain minor amounts of wetting agents, emulsifying agents, or pH buffering agents. Oral formulations may include standard carriers such as drug grade mannitol, lactose, starch, magnesium stearate, sodium saccharin, cellulose, magnesium carbonate, etc. Examples of suitable pharmaceutically acceptable carriers are described in Remington's Pharmaceutical Sciences (1990).

The pharmaceutical composition of the present invention can act systemically and/or locally. For this purpose, they can be administered by suitable routes, such as by injection, intravenously, intra-arterially, subcutaneously, intraperitoneally, intramuscularly, or transdermally; or orally, buccally, nasally, transmucosally, topically, in the form of ophthalmic formulation, or by inhalation.

The pharmaceutical composition of the present invention can be administered in suitable dosage forms for these administration routes.

The dosage forms include, but are not limited to, liquid preparations, semi-solid preparations, and solid preparations. Solid or semi-solid preparations include, but are not limited to, capsules, tablets, pills, lozenges, dragees, granules, powders, ointments, and creams. Liquid preparations include, but are not limited to, elixirs, syrups, emulsions, dispersions, suspensions, solutions, sprays, and drops.

As used herein, the term "therapeutically effective amount" refers to an amount of a compound that, when administered, will to some extent alleviate one or more symptoms of the disease or disorder being treated.

The dosing regimen may be adjusted to provide the optimal desired response. For example, a single bolus may be administered, several divided doses may be administered over time, or the dose may be reduced or increased proportionally as indicated by the exigencies of the situation of therapy. Of note is that the amount of the dosage may vary with the type and severity of the condition to be alleviated and may include single or multiple doses. It should be further understood that for any particular subject, the specific dosing regimen should be adjusted over time according to the subject's need and the professional judgment of the person administering or supervising the administration of the composition.

The amount of the compound of the present invention administered will depend on the subject being treated, the severity of the disorder or condition, the rate of administration, the disposition of the compound, and the judgment of the prescribing physician. Generally, an effective dose is about 0.0001 to about 50 mg per kg body weight per day, for example, about 0.01 to about 10 mg/kg/day (as a single or divided dose). For a 70 kg person, this will amount to about 0.007 mg/day to about 3500 mg/day, such as about 0.7 mg/day to about 700 mg/day. In some cases, a dose level not more than the lower limit of the aforementioned range may be sufficient, while in other cases, a larger dose may still be employed without causing any harmful side effects, provided that the larger dose is first divided into several smaller doses for administration throughout the day.

The content or amount of the compound of the present invention in the pharmaceutical composition can be about 0.01 mg to about 1000 mg.

As used herein, the term "treatment" means reversing, alleviating, suppressing a disease or disorder to which the term applies, or the progression of one or more symptoms of the disease or disorder. As used herein, the term "prevention" means preventing or deterring the development of, or the appearance of one or more symptoms of a disease or disorder to which the term applies.

As used herein, "subject" includes humans or non-human animals. Exemplary human subjects include human subjects suffering from a disease, e.g., the diseases described herein (referred to as patients) or normal subjects. "Non-human animals" in the present invention include all vertebrates, such as non-mammals (e.g., birds, amphibians, reptiles) and mammals, such as non-human primates, livestocks and/or domestic animals (e.g., sheep, dogs, cats, cows, pigs, etc.).

### III. Uses and Treatment Methods

The crystalline form of Compound I of the present invention has excellent GLP-1 receptor agonistic activity and can treat and/or prevent GLP-1 receptor mediated diseases and related disorders.

Therefore, in one aspect, the present invention provides a method for treating and/or preventing GLP-1 receptor mediated diseases or related disorders, comprising administering an effective amount of the crystalline form of Compound I of the present invention or the pharmaceutical composition of the present invention to a subject in need thereof.

In another aspect, the present invention provides the crystalline form of Compound I of the present invention or the pharmaceutical composition of the present invention for use in treating and/or preventing GLP-1 receptor mediated diseases and related disorders.

In another aspect, the present invention provides the use of the crystalline form of Compound I of the present invention or the pharmaceutical composition of the present invention in the manufacture of a medicament for treating and/or preventing GLP-1 receptor mediated diseases or related disorders.

In another aspect, the present invention provides a method for treating and/or preventing metabolism related diseases or disorders, comprising administering an effective amount of the crystalline form of Compound I of the present invention or the pharmaceutical composition of the present invention to a subject in need thereof.

In another aspect, the present invention provides the crystalline form of Compound I of the present invention or the pharmaceutical composition of the present invention for use in treating and/or preventing metabolism related diseases or disorders.

In another aspect, the present invention provides the use of the crystalline form of Compound I of the present invention or the pharmaceutical composition of the present invention in the manufacture of a medicament for treating and/or preventing metabolism related diseases or disorders.

In some embodiments, the metabolism related diseases or disorders include GLP-1 receptor mediated diseases and related disorders.

In some embodiments, the GLP-1 receptor mediated diseases or related disorders are selected from diabetes, hyperglycemia, insulin resistance, impaired glucose tolerance, diabetic nephropathy, diabetic neuropathy, diabetic retinopathy, adipocyte dysfunction, obesity, non-alcoholic fatty liver disease, dyslipidemia, and hyperinsulinemia.

In some embodiments, the diabetes is selected from T1D and/or T2DM, idiopathic T1D, early-onset T2DM, latent autoimmune diabetes, atypical diabetes in adolescents, and gestational diabetes.

In some embodiments, the GLP-1 receptor mediated disease or related disorder is obesity.

In some embodiments, the GLP-1 receptor mediated disease or related disorder is T2DM.

In some embodiments, the GLP-1 receptor mediated disease or related disorder is non-alcoholic fatty liver disease.

In some embodiments, the crystalline form of Compound I is selected from Crystal Form A, Crystal Form B, Crystal Form C, Crystal Form D, Crystal Form E, Crystal Form F, Crystal Form G, Crystal Form H, Crystal Form Ix, Crystal Form J, Crystal Form K, Crystal Form L, Crystal Form M, Crystal Form N, Crystal Form O, and Crystal Form P as described above. Preferably, the crystalline form of Compound I is selected from Crystal Form A, Crystal Form C, Crystal Form D, Crystal Form E, Crystal Form G, Crystal Form Ix, Crystal Form J, Crystal Form K, Crystal Form L, Crystal Form M, Crystal Form N, and Crystal Form O. More preferably, the crystalline form of Compound I is the Crystal Form A or Crystal Form G, and even more preferably the Crystal Form G.

### Beneficial Effects

The crystalline forms of Compound I of the present invention, preferably Crystal Form A and Crystal Form G, have good physical and chemical properties, such as low hygroscopicity and good physicochemical stability (solid-state stability, high-temperature resistance, high-humidity resistance, and/or high-pressure resistance). The Crystal Form G has higher thermodynamic stability at a temperature ranging from room temperature to 50°C. The Crystal Form G also has higher pressure stability. The crystalline forms of Compound I have the potential to have good drugability and/or bioavailability.

### Examples

The present invention is further elaborated through the following examples. The examples of the present invention are only used to illustrate the technical solutions of the present invention and are not intended to limit the scope of the present invention. Those skilled in the art can make some non-substantial improvements and adjustments, which still fall within the protection scope of the present invention.

All solvents used in the examples are commercially available and can be used without further purification.

The abbreviations used in this application have the meanings as follows: r.t. represents room temperature; H₂O represents water; CH₂Cl₂ represents dichloromethane; THF represents tetrahydrofuran; IPA represents isopropyl alcohol; 2-MeTHF represents 2-methyltetrahydrofuran; NMP represents N-methylpyrrolidone; DME represents dimethoxyethane; DCM represents dichloromethane; Xphos represents 2-dicyclohexylphosphino-2',4',6'-triisopropylbiphenyl; EtOAc represents ethyl acetate; MeOH represents methanol; 2-Me-THF represents 2-methyltetrahydrofuran; DAST represents diethylaminosulfur trifluoride; TFE represents tetrafluoroethylene; ACN represents acetonitrile; CPME represents cyclopentyl methyl ether; DMSO represents dimethyl sulfoxide; EtOH represents ethanol; TFA represents trifluoroacetic acid; TsOH represents p-toluenesulfonic acid; MIBK represents methyl isobutyl ketone; HEP represents n-heptane; IPAc represents isopropyl acetate; EtOAc represents ethyl acetate; DMF represents N,N-dimethylformamide; MTBE represents methyl tert-butyl ether; MEK represents methyl ethyl ketone; CHCl₃ represents chloroform; MCH represents methylcyclohexane.

Compounds are named manually or by ChemDraw^{®} software, and commercially available compounds adopt the names in the supplier catalog.

The instruments and parameters used in this application are as follows:

### 1. X-ray Powder Diffractometer (XRPD)

**Table 1: XRPD Test Parameters**

| X-ray Powder Diffractometer (XRPD) | |
|---|---|
| Model | Bruker D8 Advance |
| Serial Number | CA312 |
| Technical Specifications | Cu Kα irradiation at a wavelength of 1.54 Å (40 kV, 40 mA), θ-2θ goniometer, nickel filter, SSD160-2 detector |
| Acquisition Software | DIFFRAC.MEA.CENTER |
| Calibration Substance | Corundum (Al₂O₃) |
| c Software | MDI Jade 6 |
| Detection Angle | 3-40° 2θ |
| Step Size | 0.02° 20 |
| Speed | 0.1 s/step |
| Amount of Test Sample | >1 mg |
| Remarks | Unless otherwise specified, the samples are not ground before detection. |

### 2. Thermal Gravimetric Analyzer (TGA) and Differential Scanning Calorimeter (DSC)

**Table 2: TGA and DSC Test Parameters**

| | | | |
|---|---|---|---|
| Instruments | | METTLER TOLEDO TGA 2 | METTLER TOLEDO DSC 3 |
| | Serial Number | CA201 | CA200 |
| | Control Software | STARe Software | STARe Software |
| | Analystic Software | STARe Software | STARe Software |
| | Sample Pan | Alumina Crucible | Aluminum Crucible (with perforated Lid) |
| Parameters | Sample amount for Detection | 1 mg - 10 mg | 0.5 mg - 5 mg |
| | Protective Gas | Nitrogen | Nitrogen |
| | Gas Flow Rate | 50 mL/min | 50 mL/min |
| | General Detection Method | Segment 1 | |
| | | Initial Temperature: 30.0°C | Segment 1 |
| | | Final Temperature: 350.0°C | Initial Temperature: 30°C |
| | | | Final Temperature: 300°C |
| | | Heating Rate: 10.0 K/min | Heating Rate: 10.0 K/min |

### 3. High-Performance Liquid Chromatography (HPLC)

**Table 3: HPLC Test Parameters**

| | | | |
|---|---|---|---|
| Instrument | High Performance Liquid Chromatography | | |
| Chromatographic Column | Agilent Poroshell 120 EC-C18, 150mm * 4.6mm, 4µm | | |
| Mobile Phase | Mobile Phase A: 10 mM aqueous ammonium acetate Solution, adjusted to pH 5.0 with acetic acid Mobile Phase B: methanol/acetonitrile (3:2, v:v) | | |
| Flow Rate | 1.2 mL/min | | |
| Acquisition Time | 45 min | | |
| Determination Wavelength | 220 nm | | |
| Column Temperature | 40°C | | |
| Injector Temperature | Room Temperature | | |
| Sample Concentration and Injection Volume | Purity Determination: 1.0 mg/mL, 10 µL Equilibrium Solubility Determination: 5 µL | | |
| Diluents | Purity Determination: acetonitrile/water (1:4, v:v) Equilibrium Solubility Determination: acetonitrile/water (1:1, v:v) | | |
| Gradient Elution Schedule | Time (min) | A% | B% |
| | 0 | 60 | 40 |
| | 3 | 60 | 40 |
| | 25 | 30 | 70 |
| | 30 | 10 | 90 |
| | 35 | 10 | 90 |
| | 36 | 60 | 40 |
| | 45 | 60 | 40 |

### 4. Dynamic Vapor Sorption (DVS) Determination

**Table 4: DVS Test Parameters**

| Dynamic Vapor Sorption Instrument (DVS) | |
|---|---|
| Model | Intrinsic PLUS |
| Serial Number | DVS-001 (CA357) |
| Method and Parameters | 1. Equilibrate at 25°C |
| | 2. Humidity 0% |
| | 3. Isothermal for 360 min |
| | 4. Abort next iso if weight (%)<0.0200 |
| | 5. Step humidity 0% to 90% |
| | 6. Abort next iso if weight (%)<0.0200 |
| | 7. Step humidity 90% to 0% |

### 5. Proton Nuclear Magnetic Resonance (¹H NMR)

**Table 5: ¹H NMR Test Parameters**

| Nuclear Magnetic Resonance Instrument (NMR) | | | | |
|---|---|---|---|---|
| Instruments | Model | Bruker AVANCE III 400 MHz | Bruker AVANCE NEO 400 MHz | Bruker AVANCE NEO 400 MHz |
| | Serial Number | NMR-002 | NMR-003 | NMR-004 |
| | Detection Type \| | Proton Nuclear Magnetic Resonance Spectrum | Proton Nuclear Magnetic Resonance Spectrum | Proton Nuclear Magnetic Resonance Spectrum |
| Parameters | Full-frequency excitation, single-pulse with a 20 ppm bandwidth, excitation at a 30° angle and 8 scans, digital orthogonal detection, temperature controlled at 298K, and MeOD-d6 as the solvent. | | | |

### Example 1: Preparation of Compound 1

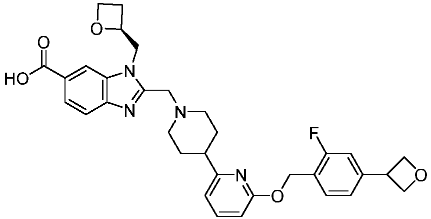

### Synthetic Scheme

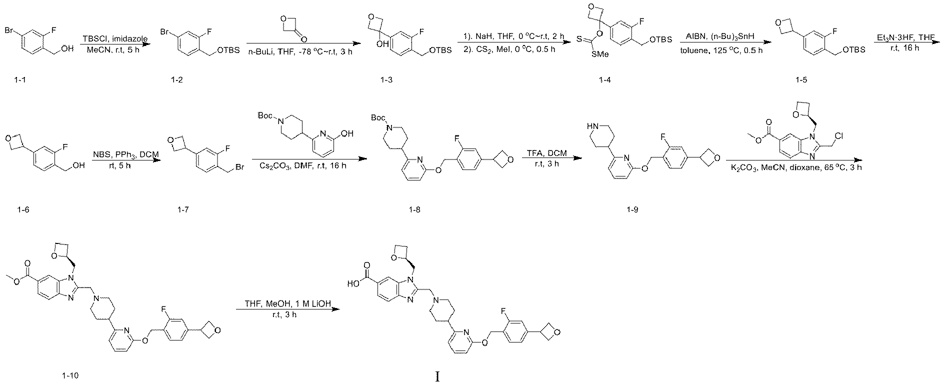

### Preparation method

Compound 1-2: To a solution of Compound 1-1 (20.0 g, 98.0 mmol) in MeCN (500 mL) was added imidazole (10.0 g, 147.0 mmol) followed by addition of TBSCl (16.3 g, 107.8 mmol). The mixture was stirred at room temperature for 5 h. H₂O (500 mL) was added. The reaction solution was extracted with EtOAc (3 × 500 mL). The combined organic phases were washed with brine (500 mL), dried (Na₂SO₄), filtered, and concentrated, and subjected to flash chromatography (SiO₂, hexane) to give 31 g of compound 1-2. Yield: 99.6%. ¹H NMR (400 MHz, DMSO-d6) δ 7.35 (m, 3H), 4.62 (s, 2H), 0.81 (s, 9H), 0.00 (s, 6H).

Compound 1-3: To a solution of Compound 1-2 (20.0 g, 62.8 mmol) in anhydrous THF (200 mL) was added dropwise N-BuLi (2.5 M in THF, 27.6 mL, 69.1 mmol) at -78°C under N₂. The mixture was stirred at this temperature for 0.5 h, and then added with oxetane-3-one (4.5 g, 62.8 mmol). The mixture was then stirred at room temperature under N₂ atmosphere for 2.5 h. The reaction solution was quenched with water (100 mL), and extracted with EtOAc (3 × 100 mL). The combined organic phases were washed with brine (100 mL), dried (Na₂SO₄), filtered, and concentrated, and subjected to flash chromatography (SiO₂, 25% EtOAc-hexane) to give 14 g of compound 1-3. Yield: 71.0%. ¹H NMR (400 MHz, DMSO-d6) δ 7.42 - 7.33 (m, 2H), 7.26 - 7.18 (m, 1H), 6.36 (s, 1H), 4.69 - 4.53 (m, 6H), 0.81 (s, 9H), -0.00 (s, 6H).

Compound 1-4: To a solution of Compound 1-3 (14.0 g, 44.8 mmol) in anhydrous THF (200 mL) was added NaH (3.6 g, 89.7 mmol) at 0°C. The mixture was stirred at room temperature for 2 h, and then added with CS₂ (3.6 g, 89.7 mmol) and MeI (6.4 g, 44.8 mmol) at 0°C under N₂. The mixture was then stirred at 0°C in N₂ for 0.5 h. The reaction solution was quenched with saturated NH₄Cl solution (100 mL), and extracted with EtOAc (3 × 200 mL). The combined organic phases were washed with brine (200 mL), dried (Na₂SO₄), filtered, and concentrated to give 14 g of compound 1-4. The product was used directly in the next step without further purification.

Compound 1-5: To a solution of Compound 1-4 (14.0 g, 44.8 mmol) in toluene (200 mL) was added (n-Bu)₃SnH (26.2 g, 89.7 mmol) followed by addition of AIBN (736 mg, 4.4 mmol). The mixture was stirred at 125°C under N₂ atmosphere for 0.5 h. The reaction solution was concentrated, and purified by flash chromatography (SiO₂, 20% EtOAc-hexane) to give 8 g of compound 1-5. Two-step yield: 60.6%. ¹H NMR (400 MHz, DMSO-d6) δ 7.41 (t, *J* = 8.0 Hz, 1H), 7.23 - 7.16 (m, 2H), 4.91 (dd, *J* = 8.3, 5.9 Hz, 2H), 4.72 (s, 2H), 4.59 (t, *J* = 6.3 Hz, 2H), 4.30 - 4.18 (m, 1H), 0.88 (s, 9H), 0.07 (s, 6H).

Compound 1-6: To a solution of Compound 1-5 (8.0 g, 43.0 mmol) in THF (200 mL) was added Et₃N·HF₃ (13.9 g, 86.0 mmol). The reaction solution was stirred at room temperature under N₂ atmosphere for 16 h. The reaction solution was concentrated, and purified by flash chromatography (SiO₂, EtOAc-hexane) to give 5 g of compound 1-6. Yield: 99.9%. ¹H NMR (400 MHz, DMSO-d6) δ 7.44 (t, *J* = 7.8 Hz, 1H), 7.20 (t, *J* = 9.1 Hz, 2H), 5.22 (t, *J* = 5.7 Hz, 1H), 4.92 (dd, *J* = 8.0, 6.1 Hz, 2H), 4.59 (t, *J* = 6.3 Hz, 2H), 4.52 (d, *J* = 5.6 Hz, 2H), 4.30 - 4.18 (m, 1H).

Compound 1-7: To a solution of Compound 1-6 (4.8 g, 26.3 mmol) in DCM (100 mL) was added NBS (5.2 g, 29.0 mmol) followed by addition of PPh₃ (7.7 g, 29.0 mmol) at 0°C. The mixture was stirred at room temperature under N₂ atmosphere for 5 h. H₂O (100 mL) was added. The reaction solution was extracted with DCM (3 × 100 mL). The combined organic phases were washed with brine (100 mL), dried (Na₂SO₄), filtered, and concentrated, and purified by flash chromatography (SiO₂, EtOAc-hexane) to give 2 g of compound 1-7. Yield: 30.7%. ¹H NMR (400 MHz, DMSO-d6) δ 7.53 (t, *J* = 8.0 Hz, 1H), 7.33 - 7.20 (m, 2H), 4.92 (dd, *J* = 8.3, 6.0 Hz, 2H), 4.70 (s, 2H), 4.60 (t, *J* = 6.3 Hz, 2H), 4.34 - 4.20 (m, 1H).

Compound 1-8: Compound 1-7 (600 mg, 2.45 mmol) and tert-butyl 4-(6-hydroxypyridin-2-yl)piperidine-1-carboxylate (684 mg, 2.45 mmol) were added to the solvent DMF (50 mL). Then Cs₂CO₃ (2.4 g, 7.37 mmol) was added. The reaction solution was stirred at room temperature for 16 h. H₂O (50 mL) was added. The reaction solution was extracted with EtOAc (3 × 50 mL). The combined organic phases were washed with brine (50 mL), dried (Na₂SO₄), filtered, and concentrated, and purified by flash chromatography (SiO₂, EtOAc-hexane) to give 500 mg of compound 1-8. Yield: 45.9%. ¹H NMR (400 MHz, CDCl₃) δ 7.60 (t, *J* = 7.7 Hz, 1H), 7.46 (t, *J* = 7.6 Hz, 1H), 7.17 (s, 1H), 7.13 (d, *J =* 11.4 Hz, 1H), 6.75 (d, *J* = 7.3 Hz, 1H), 6.68 (d, *J* = 8.1 Hz, 1H), 5.43 (d, *J* = 7.5 Hz, 3H), 5.33 (s, 1H), 4.45 (s, 2H), 4.14 (d, *J* = 14.0 Hz, 2H), 3.03 (t, *J* = 12.8 Hz, 1H), 2.80 (t, *J* = 12.9 Hz, 2H), 1.85 (d, *J* = 12.5 Hz, 2H), 1.57-1.61 (m 3H), 1.42 (s, 9H). / LC-MS (ESI) m/z: 443.2 [M⁺H]⁺.

Compound 1-9: To a solution of Compound 1-8 (210 mg, 0.49 mmol) in DCM (10 mL) was added TFA (10 mL). The reaction solution was stirred at room temperature for 3 h. The reaction solution was concentrated to give 250 mg of compound 1-9. LC-MS: MC20-1128-086C (ESI) m/z: 343.1 [M⁺H]⁺.

Compound 1-10: Compound 1-9 (200 mg, 0.58 mmol) and (S)-methyl 2-(chloromethyl)-1-(oxetan-2-ylmethyl)-1H-benzo[d]imidazole-6-carboxylate (172 mg, 0.58 mmol) were added to the solvents dioxane (20 mL) and MeCN (12 mL), followed by K₂CO₃ (162 mg, 1.16 mmol). The reaction solution was stirred at 65°C for 3 h. H₂O (20 mL) was added. The reaction solution was extracted with EtOAc (3 × 20 mL). The combined organic phases were washed with brine (20 mL), dried (Na₂SO₄), filtered, and concentrated, and purified by flash chromatography (SiO₂, EtOAc-hexane) to give 60 mg of compound 1-10. Yield: 22.0%. ¹H NMR (400 MHz, DMSO-d6) δ 8.30 (d, *J* = 1.1 Hz, 1H), 7.82 (dd, *J* = 8.5, 1.6 Hz, 1H), 7.70 - 7.59 (m, 2H), 7.53 (t, *J* = 7.8 Hz, 1H), 7.27 (d, *J =* 11.3 Hz, 1H), 7.21 (d, *J =* 9.5 Hz, 1H), 6.86 (d, *J* = 7.4 Hz, 1H), 6.65 (d, *J* = 8.0 Hz, 1H), 5.38 (s, 2H), 5.35 - 5.30 (m, 1H), 5.12 (qd, *J* = 7.0, 2.5 Hz, 1H), 4.90 (dd, *J* = 8.3, 6.0 Hz, 2H), 4.80-4.84 (m 1H), 4.65-4.71 (m, 1H), 4.58 (t, *J* = 6.4 Hz, 2H), 4.47 (dt, *J* = 8.3, 6.5 Hz, 1H), 4.37 (dt, *J =* 9.1, 5.9 Hz, 1H), 4.21-4.28 (m,1H), 3.94-4.02 (m,1H), 3.87 (s, 3H), 3.78 (d, *J =* 13.6 Hz, 1H), 3.01 (d, *J* = 9.4 Hz, 1H), 2.85 (d, *J* = 13.5 Hz, 1H), 2.73 - 2.59 (m, 2H), 2.27 (d, *J* = 10.0 Hz, 1H), 2.17 (d, *J* = 11.6 Hz, 1H), 1.76 (m, 4H). / LC-MS (ESI) m/z: 601.4 [M⁺H]⁺.

Compound 1: To a solution of Compound 1-10 (60 mg, 0.1 mmol) in MeOH (1 mL) and THF (5 mL) was added 1 M LiOH (2 mL). The reaction solution was stirred at room temperature for 3 h. The reaction solution was concentrated, and purified by preparative HPLC to give 10.95 mg of compound 1 as a white solid. Yield: 18.6%. ¹H NMR (400 MHz, DMSO-d6) δ 8.20 (s, 1H), 7.79 (dd, J₁ = 4.0 Hz, , J₂ = 8.0 Hz, 1H), 7.62 (t, J = 8.0 Hz, 1H), 7.54 (t, J = 8.0 Hz, 1H), 7.45 (d, J = 8.0 Hz, 1H), 7.27 (d, J = 12.0 Hz, 1H), 7.21 (d, J = 8.0 Hz, 1H), 6.87 (d, J = 8.0 Hz, 1H), 6.65 (d, J = 8.0 Hz, 1H), 5.38 (s, 2H), 5.12 (m, 1H), 4.90 (dd, J = 8.0 Hz, 2H), 4.77 (dd, J₁ = 4.0 Hz, J₂ = 16.0 Hz, 1H), 4.64 (d, J = 4.0 Hz, 1H), 4.58 (m, 2H), 4.50 - 4.44 (m, 1H), 4.38 (m, 1H), 4.29 - 4.19 (m, 1H), 3.94 (d, J = 12.0 Hz, 1H), 3.77 (d, J = 12.0 Hz, 1H), 3.00 (d, J = 12.0 Hz, 1H), 2.86 (d, J = 12.0 Hz, 1H), 2.71 (m, 1H), 2.64 - 2.56 (m, 1H), 2.47 - 2.42 (m, 1H), 2.21 (m, 2H), 1.73 (m, 4H).

Compound I was in an amorphous form, as determined by XRPD, with an XRPD pattern as shown in Figure 1.

### Example 2 - Assay of GLP-1R agonistic activity of Compound I

### (1) Test instruments and reagents

**Table 6: Instruments and Reagents for Biological Activity Tests**

| Instruments/reagents | Supplier | Model |
|---|---|---|
| cAMP-GS DYNAMIC kit | CisBio | 62AM4PEC |
| DMEM | CellMax | CGN101.5 |
| FBS | Gemini | 900-108 |
| 1% Pen-3trep | Sangom biotech | E607011-0100 |
| IBMX | Meilunbio | MB5226 |
| 384 well plate | Corning | 3824 |
| Incubator | Thermo | 3111 |
| Microscope | Jiangnan | XD-202 |
| Cell counter | Counter Star | Star IC1000 |
| Plate reader | Tecan | Tecan Spark |

### (2) GLP-1R kit

GLP-1R-mediated agonist activity was determined by cell-based assays using a homogeneous time-resolved fluorescence (i.e., HTRF)-based cAMP detection kit, which measures the level of cAMP in cells. The method was a competitive immunoassay. It enabled direct pharmacological characterization of compounds acting on Gs-coupled receptors in adherent or suspending cells.

The standard curve of native cAMP or unlabeled cAMP produced by cells competed with d2-labeled cAMP red receptors to bind monoclonal anti-cAMP Eu3+ cryptate donors, and the specific signal was inversely proportional to the concentration of cAMP in standard or tested samples.

Human GLP-1R encoding sequence (NCBI reference sequence NP_002053.3) was subcloned into pEGFP-N1 (tsingke), and the cell line stably expressing the receptor was isolated. The expression density of GLP-1R was confirmed by the expression of GFP observed under a fluorescence microscope.

### (3) GLP-1R-GFP-293A cell culture

293A GFP-GLP-1R cells were incubated in DMEM growth medium, 10% heat-inactivated fetal bovine serum (GEMINI Cat # 900-108), 1% Pen-3Trep (Sangom Biotech Cat # E607011-0100)] in a moist incubator with 5% CO₂ at 37 °C.

### (4) cAMP level test method

The tested compound (in DMSO) at different concentrations was 1:5 diluted in distilled water in a stimulating buffer, followed by addition of 500 µm 3-isobutyl-1-methylxanthine (IBMX; Meilunbiocat # MB5226) to obtain a working solution of 2X compound, and then 5 µL of the compound was added to a white 384-well assay plate (Corning 3824) using a multichannel pipette. The final DMSO concentration in the buffer mixture was determined to be 1 ‰.

Cells were collected from a T25 tissue culture flask and centrifuged at room temperature at 1000 rpm for 5 minutes. The cell precipitates were then re-suspended in 1 mL of the stimulating buffer. 20 µL sample of cell suspension was counted on a counter STAR IC 1000 to determine the cell viability and the cell count per mL. The remaining cell suspension was then regulated with the stimulating buffer to deliver 2000 living cells per well using a multichannel pipette. 5 µL of the cell suspension was added to each well of the plate which already contained the compound. The plate was sealed and incubated at 37 °C with 5% CO₂ for 30 minutes.

After 30 minutes of incubation, 5 µL of d2-labeled cAMP and 5 µL of anti-cAMP cryptate (both 1: 20 diluted in the cell lysis buffer) were added to each well of the plate. The plate was then incubated at room temperature for 60 minutes, and the changes of HTRF signal were read with Tecan Spark reader: absorbance values at 340 nm (excitation)/at 615 nm and 665 nm (emission). Raw data were converted into nM cAMP by interpolation from the cAMP standard curve, and the effect in percentage was determined relative to the saturated concentration of the complete agonist GLP-17~37 (400 nM) contained in each plate. Determination of EC50 was performed based on the agonist dose-response curve, which was analyzed using a four-parameter logical dose-response equation with a curve fitting program.

This test proved that Compound I activated GLP-1R signaling through the cAMP pathway, thus acting as a GLP-1R agonist. The test data presented the results in the form of a geometric mean (EC₅₀s) based on the number of repetition times.

Experimental results: Compound I has a strong agonistic effect on GLP-1R.

| Compound | EC₅₀ (nM) |
|---|---|
| 1 | 0.03 |

### Example 3: Preparation of Crystal Forms of Compound I

A total of 16 crystal forms were screened out from 111 polymorph screening tests with the amorphous form as the starting material, including slow evaporation, crystal slurry at 4-8°C, crystal slurry at room temperature, crystal slurry at 50°C, addition of an anti-solvent, addition of an anti-anti-solvent, cooling crystallization, gas-liquid diffusion, gas-solid diffusion, water vapor stress, polymer induction, grinding, cyclic temperature rising and falling, and rotary evaporation. Among them, 12 crystal forms described in Example 3 were stable crystal forms, comprising Crystal Form A and Crystal Form G which were anhydrous crystal forms, with the rest being crystal forms of corresponding solvates.

### 1. Crystal Form A

200.49 mg of amorphous Compound I was suspended in a 5 mL mixture of acetone/H₂O (1/4, v/v). The suspension was magnetically stirred at 50°C for about 1 day, and then the solid was separated to obtain Crystal Form A, with an XRPD pattern as shown in Figure 2-1.

### 2. Crystal Form G

199.87 mg of amorphous Compound I was suspended in 18 mL of ACN. The suspension was stirred at 50°C, then heated to 60°C to dissolve, and then filtered. The clear solution was allowed to cool naturally to room temperature, and the solid was separated, which was Crystal Form G, with an XRPD pattern as shown in Figure 3-1.

### 3. Crystal Form C

19.94 mg of Compound I was dissolved in 0.5 mL of THF and filtered. Then, 1.0 mL of H₂O was added to the clear solution under magnetic stirring. A large amount of white precipitate was formed, and the solid was separated, which was Crystal Form C, with an XRPD pattern as shown in Figure 4-1.

### 4. Crystal Form D

19.81 mg of Compound I was dissolved in 0.5 mL of 2-MeTHF and filtered. Then, 1.0 mL of HEP was added to the clear solution under magnetic stirring. A large amount of precipitate was formed, and the solid was separated, which was Crystal Form D, with an XRPD pattern as shown in Figure 5-1.

### 5. Crystal Form E

100.18 mg of amorphous Compound I was added to a 20 mL vial, and then 2.5 mL of MTBE was added. The resulting suspension was magnetically stirred at 50°C for about 1 day, and then the solid was collected by centrifugation, which was Crystal Form E, with an XRPD pattern as shown in Figure 6-1.

### 6. Crystal Form Ix

99.79 mg of amorphous Compound I was added to 4.0 mL of acetone and filtered. The vial containing the filtrate was sealed with parafilm, the parafilm was perforated, and the vial was placed at room temperature to slowly evaporate for 3 days. The obtained solid was collected, which was Crystal Form Ix, with an XRPD pattern as shown in Figure 7-1.

### 7. Crystal Form J

20.05 mg of Compound I was suspended in a 0.8 mL mixture of EtOH/MTBE (1/3 v/v). The suspension was allowed to become clear at 50°C, and then filtered. The clear solution was allowed to cool naturally to room temperature, and the solid was separated, which was Crystal Form J, with an XRPD pattern as shown in Figure 8-1.

### 8. Crystal Form K

20 mg of Compound I was suspended in a 1.2 mL mixture of CH₂Cl₂/MTBE (1/3 v/v). The suspension was allowed to become clear at 50°C, and then filtered. The clear solution was allowed to cool naturally to room temperature, and the solid was separated, which was Crystal Form K, with an XRPD pattern as shown in Figure 9-1.

### 9. Crystal Form L

100.14 mg of Compound I was suspended in a 9.0 mL mixture of THF/H₂O (1/2 v/v). The suspension was allowed to become clear at 50°C, and then filtered. The clear solution was allowed to cool naturally to room temperature, and the solid was separated, which was Crystal Form L, with an XRPD pattern as shown in Figure 10-1.

### 10. Crystal Form M

99.83 mg of Compound I was suspended in a 10 mL mixture of anisole/HEP (9/1 v/v). The suspension allowed to become clear at 50°C, and then filtered. The clear solution was allowed to cool naturally to room temperature, and the solid was separated, which was Crystal Form M, with an XRPD pattern as shown in Figure 11-1.

### 11. Crystal Form N

20.08 mg of Compound I was suspended in a 1.0 mL mixture of IPA/H₂O (4/1 v/v) and filtered. The vial containing the filtrate was sealed with parafilm, the parafilm was perforated, and the vial was placed at room temperature to slowly evaporate for 8 days. The obtained solid was collected, which was Crystal Form N, with an XRPD pattern as shown in Figure 12-1.

### 12. Crystal Form O

20.17 mg of amorphous Compound I was added to a 3 mL vial, and then this vial was placed into a 20 mL vial containing 3 mL of acetone. The 20 mL vial was sealed with a lid and placed at room temperature to allow the acetone vapor to interact with Compound I. After 10 days, the obtained solid was taken out, which was Crystal Form O, with an XRPD pattern as shown in Figure 13-1.

Compared with the metastable crystal forms described in Example 4, no weakening of crystallinity or crystal form transformation was observed in the crystal forms described in Example 3 under a dry condition at 50°C.

Anhydrous Crystal Forms A and G both had melting points over 170°C, and both showed excellent crystal form properties in terms of equilibrium solubility, hygroscopicity, solid-state stability, pressure stability, and high-humidity stability.

Crystal Form C, Crystal Form O, Crystal Form E, Crystal Form Ix, Crystal Form J, Crystal Form K, Crystal Form L, Crystal Form M, Crystal Form N, and Crystal Form D, as solvates, could remain stable for a long time, when solvent evaporation at a high-temperature (such as greater than 100°C) did not occur.

### Example 4: Preparation of Metastable Crystal Forms of Compound I

### 1. Crystal Form B

200.09 mg of amorphous Compound I was suspended in a 5 mL mixture of CH₂Cl₂/HEP (1/2 v/v). The suspension was magnetically stirred at room temperature for about 1 day, and then the solid was separated, which was Crystal Form B, with an XRPD pattern as shown in Figure 14.

### 2. Crystal Form H

100.06 mg of Compound I was suspended in an 11 mL mixture of EtOAc/HEP (2/1 v/v). Then, the suspension was heated to 50°C and equilibrated for about 2 hours, and then filtered. The filtrate was slowly cooled to room temperature in a water bath. The solid was separated to obtain Crystal Form H, with an XRPD pattern as shown in Figure 15.

### 3. Crystal Form F

100.14 mg of Compound I was added to a 9.0 mL mixture of 2-MeTHF/HEP (8/1 v/v) and filtered. The vial containing the filtrate was sealed with parafilm, the parafilm was perforated, and the vial was placed at room temperature to slowly evaporate for 3 days. The obtained solid was collected to give Crystal Form F, with an XRPD pattern as shown in Figure 16.

### 4. Crystal Form P

19.87 mg of Compound I was added to a 5.0 mL vial, and 1.0 mL of CH₂Cl₂ was added, and filtered to obtain a clear solution. This solution was added to a 20 mL vial containing 3 mL of HEP, and then the solid was separated to give Crystal Form P, with an XRPD pattern as shown in Figure 17.

The 4 metastable crystal forms described in Example 4 had lower crystal form stability than each crystal form described in Example 3. Crystal Form B would convert into an amorphous form when dried at 50°C for 3 hours. Crystal Form H showed a weak crystalline state when dried at 50°C for 2 hours. Crystal Form F transformed into stable Crystal Form D under the condition of being sealed for 21 days. Crystal Form P would convert into an amorphous form when dried at 50°C for 3.5 hours.

### Example 5: Determination of Equilibrium Solubility of Compound I

The equilibrium solubility of Crystal Form A and Crystal Form G of Compound I was determined for 24 hours at 37°C in 4 media, namely H₂O (purified water), FaSSGF (fasted-state simulated gastric fluid), FaSSIF (fasted-state simulated intestinal fluid), and FeSSIF (fed-state simulated intestinal fluid).

Crystal Form A and Crystal Form G each in an amount of 10 mg were separately added to 1 mL of a corresponding medium and magnetically stirred at a constant temperature (37±2 °C). After 24 hours, samples were taken and centrifuged for separation. The obtained solids were tested by XRPD, and the supernatants were filtered for solubility testing.

The results shown in Table E-1 indicate that after stirring in different media for 24 hours, no crystal form change was observed for Crystal Form A and Crystal Form G in H₂O, FeSSIF, and FaSSIF; and a hydrochloride salt was formed in FaSSGF. In water, the equilibrium solubility of both Crystal Form A and Crystal Form G was less than 0.1 mg/mL.

**Table E-1: Equilibrium Solubility of Crystal Form A and Crystal Form G of Compound I**

| Sample | Medium | Solubility after 24 h (mg/mL) | Crystal Form | Whether the Crystal Form Changed |
|---|---|---|---|---|
| Crystal Form A | H₂O | 0.08 | Crystal Form A | NO |
| | FaSSIF | 0.05 | Crystal Form A | NO |
| | FeSSIF | 0.32 | Crystal Form A | NO |
| | FaSSGF | 0.20 | Hydrochloride salt | YES |
| Crystal Form G | H₂O | 0.06 | Crystal Form G | NO |
| | FaSSIF | 0.08 | Crystal Form G | NO |
| | FeSSIF | 0.27 | Crystal Form G | NO |
| | FaSSGF | <0.01 | Hydrochloride salt | YES |

Crystal Form A has a significantly increased solubility in both FeSSIF and FaSSGF as compared to water, which is beneficial for pre-meal or post-meal administration in the case of oral administration. Crystal Form G has a significantly increased solubility in FeSSIF as compared to water, which is beneficial for post-meal administration in the case of oral administration. In the case of pre-meal oral administration, Crystal Form A may be more advantageous than Crystal Form G.

### Example 6: Determination of Hygroscopicity of Crystal Form A and Crystal Form G of Compound I

To evaluate the risk of stability change of the samples with humidity at 25°C, DVS tests were carried out on Crystal Form A and Crystal Form G of Compound I. The test results are summarized in Table E-2. The DVS pattern of Crystal Form A is shown in Figure 2-5, and the DVS pattern of Crystal Form G is shown in Figure 3-5.

**Table E-2: Hygroscopicity of Crystal Form A and Crystal Form G**

| Sample | Sample Weight Change at 80% RH (%) | Hygroscopicity * |
|---|---|---|
| Crystal Form A | 0.89 | Slightly hygroscopic |
| Crystal Form G | 0.73 | Slightly hygroscopic |

| | | |
|---|---|---|
| *: Hygroscopicity is based on the weight gain of the sample when the humidity rises to 80% RH at 25°C. 0.2%-2% is slightly hygroscopic, and 2%-15% is hygroscopic. | | |

The results show that under the condition of 80% RH, Crystal Form A absorbed 0.89% water and Crystal Form G absorbed 0.73% water, indicating that both were slightly hygroscopic. No crystal form change was observed in the samples before and after the DVS tests. Both Crystal Form A and G have good low hygroscopicity.

### Example 7: Determination of Solid-State Stability of Crystal Form A and Crystal Form G of Compound I

Crystal Form A and Crystal Form G of Compound I were each placed under long-term (25°C/60% RH) and accelerated (40°C/75% RH) conditions for 10 days, and then the HPLC purity and crystal form changes were determined. The test results are summarized in Table E-3.

**Table E-3: Results of Solid-State Stability Determination**

| Sample | Initial Purity (% Area) | 25°C/60%RH, 10 days | | 40°C/75%RH, 10 days | |
|---|---|---|---|---|---|
| | | Crystal form change | Purity (% Area) | Crystal form change | Purity (% Area) |
| Crystal Form A | 98.11 | NO | 97.50 | NO | 97.33 |
| Crystal Form G | 97.81 | NO | 97.37 | NO | 97.43 |

The results show that no crystal form change was observed in Crystal Form A and G before and after testing; the purity of Crystal Form A decreased by about 0.6% under long-term conditions and by about 0.8% under accelerated conditions; the purity of Crystal Form G decreased by about 0.4% under long-term conditions and by about 0.4% under accelerated conditions. Both Crystal Form A and G showed good solid-state stability under long-term and accelerated conditions and there was no significant difference between them, except that Crystal Form G seemed to be less affected by the test conditions.

### Example 8: Determination of High-Temperature Stability of Crystal Form A and Crystal Form G of Compound I

Crystal Form A and Crystal Form G of Compound I were each placed at 60°C for 1 day and 10 days, and then the HPLC purity and crystal form changes were tested. The test results are summarized in Table E-4.

**Table E-4: Results of High-Temperature Stability Determination**

| Sample | Initial Purity (% Area) | 60°C, 1 day | | 60°C, 10 days | |
|---|---|---|---|---|---|
| | | Crystal form change | Purity (% Area) | Crystal form change | Purity (% Area) |
| Crystal Form A | 98.11 | NO | 98.00 | NO | 96.98 |
| Crystal Form G | 97.81 | NO | 97.55 | NO | 96.63 |

The results show that the purity of Crystal Form A decreased by about 0.1% after 1 day at the high temperature and by about 1.1% after 10 days at the high temperature; and the purity of Crystal Form G decreased by about 0.3% after 1 day at the high temperature and by about 1.2% after 10 days at the high temperature. No crystal form change was observed in all samples. Both Crystal Forms A and G showed good high-temperature stability, and there was no significant difference between them.

### Example 9: Determination of High-Humidity Stability of Crystal Form A and Crystal Form G of Compound I

Crystal Form A and Crystal Form G of Compound I were each placed under high-humidity (90% RH) conditions for 1 day and 10 days, and then the HPLC purity and crystal form changes were tested. The test results are summarized in Table E-5.

**Table E-5: Results of High-Humidity Stability Determination**

| Sample | Initial Purity (% Area) | 90% RH, 1 Day | | 90% RH, 10 Days | |
|---|---|---|---|---|---|
| | | Crystal form change | Purity (% Area) | Crystal form change | Purity (% Area) |
| Crystal Form A | 98.11 | NO | 97.77 | NO | 97.07 |
| Crystal Form G | 97.81 | NO | 97.67 | NO | 96.53 |

The results show that the purity of Crystal Form A decreased by about 0.3% after 1 day under the high humidity and by about 1.0% after 10 days under the high humidity; the purity of Crystal Form G decreased by about 0.1% after 1 day under the high humidity and by about 1.3% after 10 days under the high humidity. No crystal form change was observed in all samples. Both Crystal Form A and G showed good high-humidity stability, and there was no significant difference between them.

### Example 10: Determination of Pressure Stability of Crystal Form A and Crystal Form G of Compound I

Crystal Form A and Crystal Form G of Compound I were each pressed at a pressure of 1000 Mpa for 5 minutes, and then the HPLC purity and crystal form changes were tested. The test results are summarized in Table E-6.

**Table E-6: Results of Pressure Stability Determination**

| Sample | Initial Purity (% Area) | 1000 Mpa, 5 min | |
|---|---|---|---|
| | | Crystal form change | Purity (% Area) |
| Crystal Form A | 98.11 | NO | 97.44 |
| Crystal Form G | 97.81 | NO | 97.65 |

The results show that the purity of Crystal Form A decreased by about 0.7% and the purity of Crystal Form G decreased by about 0.2%. No crystal form change was observed in all samples. The pressure stability of Crystal Form G was better than that of Crystal Form A.

Examples herein are used to illustrate the principles and implementation methods of the present invention. The Examples above are merely for helping understand the present invention without any limitation thereto. Of noted is that for those of ordinary skill in the art, several improvements and modifications can be made to the present invention without departing from the principle of the present invention, and these improvements and modifications also fall within the scope of the claims of the present invention.

## Claims

1. A crystalline form of Compound I, i.e., (S)-2-((4-(6-((2-fluoro-4-(oxetan-3-yl)benzyl) oxy)pyridin-2-yl)piperidin-1-yl)methyl)-1-(oxetan-2-ylmethyl)-1H-benzo[d]imidazole-6-carboxylic acid.

2. The crystalline form according to claim 1, wherein the crystalline form is Crystal Form A, wherein:
the X-ray powder diffraction (XRPD) pattern of the Crystal Form A includes diffraction peaks at the following diffraction angles (2θ): about 4.97 ± 0.2°, 11.74 ± 0.2°, 13.27 ± 0.2°, 21.04 ± 0.2°, and 24.06 ± 0.2°; and/or
the differential scanning calorimetry (DSC) pattern of the Crystal Form A has one endothermic peak with a peak value at about 177.8°C ± 3.0 °C; and/or
the Crystal Form A has a weight loss of about 0.037% during the process of heating to about 150°C ± 3 °C, as measured by thermogravimetric analysis (TGA); and/or
the ¹H NMR spectrum of the Crystal Form A is substantially as shown in Figure 2-4;
preferably, the XRPD pattern of the Crystal Form A further includes diffraction peaks at any one, more, or all of the following diffraction angles (2θ): about 15.88 ± 0.2°, 16.26 ± 0.2°, 16.44 ± 0.2°, 18.43 ± 0.2°, 19.17 ± 0.2°, 19.48 ± 0.2°, 19.65 ± 0.2°, 19.89 ± 0.2°, 20.11 ± 0.2°, 20.67 ± 0.2°, 21.27 ± 0.2°, 22.81 ± 0.2°, and 23.4 ± 0.2°;
more preferably, the XRPD pattern of the Crystal Form A further includes diffraction peaks at any one, more, or all of the following diffraction angles (2θ): about 9.99 ± 0.2°, 17.65 ± 0.2°, 20.32 ± 0.2°, 24.26 ± 0.2°, 24.76 ± 0.2°, 25.79 ± 0.2°, 26.75 ± 0.2°, 27.47 ± 0.2°, 28.19 ± 0.2°, 29.38 ± 0.2°, and 29.91 ± 0.2°; and/or
the DSC pattern of the Crystal Form A is substantially as shown in Figure 2-2; and/or
the TGA pattern of the Crystal Form A is substantially as shown in Figure 2-3;
more preferably, the XRPD pattern of the Crystal Form A includes diffraction peaks at diffraction angles (2θ) that are substantially the same as those shown in Figure 2-1, and further more preferably, the XRPD pattern of the Crystal Form A is as shown in Figure 2-1;
more preferably, the Crystal Form A is not a solvate, and more preferably is an anhydrate.

3. The crystalline form according to claim 1, wherein the crystalline form is Crystal Form G, wherein:
the XRPD pattern of the Crystal Form G includes diffraction peaks at the following diffraction angles (2θ): about 4.99 ± 0.2°, 10.05 ± 0.2°, 16.99 ± 0.2°, 19.21 ± 0.2°, and 20.28 ± 0.2°; and/or
the DSC pattern of the Crystal Form G has one endothermic peak with a peak value at about 188.3°C ± 3.0 °C; and/or
the Crystal Form G has a weight loss of about 0.494% during the process of heating to about 180°C ± 3 °C, as measured by TGA;
preferably, the XRPD pattern of the Crystal Form G further includes diffraction peaks at any one, more, or all of the following diffraction angles (2θ): about 12.33 ± 0.2°, 15.84 ± 0.2°, 17.34 ± 0.2°, 17.59 ± 0.2°, 20.67 ± 0.2°, 21.68 ± 0.2°, and 26.59 ± 0.2°;
more preferably, the XRPD pattern of the Crystal Form G further includes diffraction peaks at any one, more, or all of the following diffraction angles (2θ): about 12.62 ± 0.2°, 15.00 ± 0.2°, 18.30 ± 0.2°, 23.88 ± 0.2°, 24.72 ± 0.2°, 29.12 ± 0.2°, and 33.76 ± 0.2°; and/or
the DSC pattern of the Crystal Form G is substantially as shown in Figure 3-2; and/or
the TGA pattern of the Crystal Form G is substantially as shown in Figure 3-3;
more preferably, the XRPD pattern of the Crystal Form G includes diffraction peaks at diffraction angles (2θ) that are substantially the same as those shown in Figure 3-1, and further more preferably, the XRPD pattern of the Crystal Form G is as shown in Figure 3-1;
more preferably, the Crystal Form G is not a solvate, and more preferably is an anhydrate.

4. A pharmaceutical composition comprising the crystalline form according to any one of claims 1 to 3, and a pharmaceutically acceptable carrier,
wherein the crystalline form is preferably the Crystal Form A according to claim 2 or the Crystal Form according to claim 3.

5. The crystalline form according to any one of claims 1 to 3, or the pharmaceutical composition according to claim 4, for use in treating and/or preventing GLP-1 receptor mediated diseases and related disorders,
wherein the crystalline form is preferably the Crystal Form A according to claim 2 or the Crystal Form G according to claim 3.

6. Use of the crystalline form according to any one of claims 1 to 3, or the pharmaceutical composition according to claim 4 in the manufacture of a medicament for treating and/or preventing GLP-1 receptor mediated diseases or related disorders,
wherein the crystalline form is preferably the Crystal Form A according to claim 2 or the Crystal Form G according to claim 3.

7. A method for treating and/or preventing GLP-1 receptor mediated diseases or related disorders, comprising administering an effective amount of the crystalline form according to any one of claims 1 to 3, or the pharmaceutical composition according to claim 4 to a subject in need thereof,
wherein the crystalline form is preferably the Crystal Form A according to claim 2 or the Crystal Form G according to claim 3.

8. The crystalline form or pharmaceutical composition according to claim 5, the use according to claim 6, or the method according to claim 7, wherein the GLP-1 receptor mediated diseases or related disorders are selected from diabetes, hyperglycemia, insulin resistance, impaired glucose tolerance, diabetic nephropathy, diabetic neuropathy, diabetic retinopathy, adipocyte dysfunction, obesity, non-alcoholic fatty liver disease, dyslipidemia, and hyperinsulinemia;
wherein the diabetes is preferably selected from T1D and/or T2DM, idiopathic T1D, early-onset T2D, latent autoimmune diabetes, atypical diabetes in adolescents, and gestational diabetes.
